(19) Europäisches Patentamt
European Patent Office
Office européen des brevets

(11) Publication number: **0 496 595 B1**

(12) **EUROPEAN PATENT SPECIFICATION**

(45) Date of publication of patent specification: **06.09.95**

(51) Int. Cl.[6]: **C07C 311/08**, C07C 271/28, C07D 239/54, A01N 43/54

(21) Application number: **92300543.3**

(22) Date of filing: **22.01.92**

(54) **Aniline derivatives and process for producing the same.**

(30) Priority: **23.01.91 JP 6332/91**
**03.12.91 JP 319421/91**

(43) Date of publication of application:
**29.07.92 Bulletin 92/31**

(45) Publication of the grant of the patent:
**06.09.95 Bulletin 95/36**

(84) Designated Contracting States:
**AT BE CH DE DK ES FR GB GR IT LI LU MC NL PT SE**

(56) References cited:
**EP-A- 0 027 887**
**EP-A- 0 069 584**
**EP-A- 0 416 684**
**DE-A- 2 018 969**
**US-A- 3 920 444**

**JOURNAL OF AGRICULTURAL AND FOOD CHEMISTRY. vol. 22, no. 6, 1974, WASHINGTON US pages 1111 - 1119; R.D. TREPKA ET AL: 'SYNTHESIS AND HERBICIDAL ACTIVITY OF FLUORINATED N-PHENYLALKANESULFONAMIDES' PAGES 1114-1115, TABLE II, EXAMPLES 142, 147, 148.**

(73) Proprietor: **NISSAN CHEMICAL INDUSTRIES, LIMITED**
**7-1, Kanda-Nishiki-cho 3-chome**
**Chiyoda-ku**
**Tokyo 101 (JP)**

(72) Inventor: **Kawamura, Yasuo**
**1-5-16-406 Narashino**
**Funabashi-shi,**
**Chiba-ken (JP)**
Inventor: **Satow, Jun**
**1-5-17-504 Narashino**
**Funabashi-shi,**
**Chiba-ken (JP)**
Inventor: **Fukuda, Kenzou**
**3053-1 Ajisu-cho**
**Yoshiki-gun**
**Yamaguchi-ken (JP)**
Inventor: **Itoh, Kaoru**
**1-5-16-506 Narashino**
**Funabashi-shi,**
**Chiba-ken (JP)**
Inventor: **Kito, Hiroshi**
**6-13-1 Konakadai**
**Chiba-shi,**
**Chiba-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

(74) Representative: **Woods, Geoffrey Corlett**
**J.A. KEMP & CO.**
**14 South Square**
**Gray's Inn**
**London WC1R 5LX (GB)**

## Description

The present invention relates to intermediates useful for producing a 6-haloalkyl-3-phenyluracil derivative [described in JP-A-2-400475 (1990) and JP-A-3-314391 (1991)] which is useful as a herbicide and a to process for producing said intermediates and derivative.

As aniline derivatives, N-(3-amino-4-chlorophenyl)methanesulfonamide is described in DE-C-2938633 and DE-C-3124172, methyl 5-amino-2-chlorocarbanilate is described in DE-C-2703838, methyl 2-chloro-5-nitrocarbanilate is described in US-A-2860166, DE-C-2703838, DE-C-2725146, DE-C-2846625 and DE-C-2926049, ethyl 5-amino-2-chlorocarbanilate is described in US-A-3920444, ethyl 2-chloro-5-nitrocarbanilate is described in Justus Liebigs Ann. Chem. vol 721. p.14, N-(4-fluoro-3-nitrophenyl)methanesulfonamide is described in US-A-4507479, a methyl ester and a phenyl ester of 2-fluoro-5-nitrocarbanilic acid are discribed in US-A-4226613, and a methyl ester and a phenyl ester of 5-amino-2-fluorocarbanilic acid (since they are not isolated, the physical properties are not described) are described in US-A-4227007.

These references only describe these derivatives as intermediates or byproducts, and the processes for producing these derivatives are not always satisfactory. Provision of a more advantageous process is, therefore, demanded.

In addition, no benzene derivative having both carbonate and sulfoneamide functional groups and substituted by one or two halogen atom is known.

As a result of earnest investigations of a process for producing a 6-haloalkyl-3-phenyluracil derivative [described in JP-A-2-400475 (1990) and JP-A-3-314391 (1991)] which is useful as a herbicide, it has been found that intermediates obtainable in accordance with the following scheme are useful as an intermediate for producing the 6-haloalkyl-3-phenyluracil derivative:

Scheme

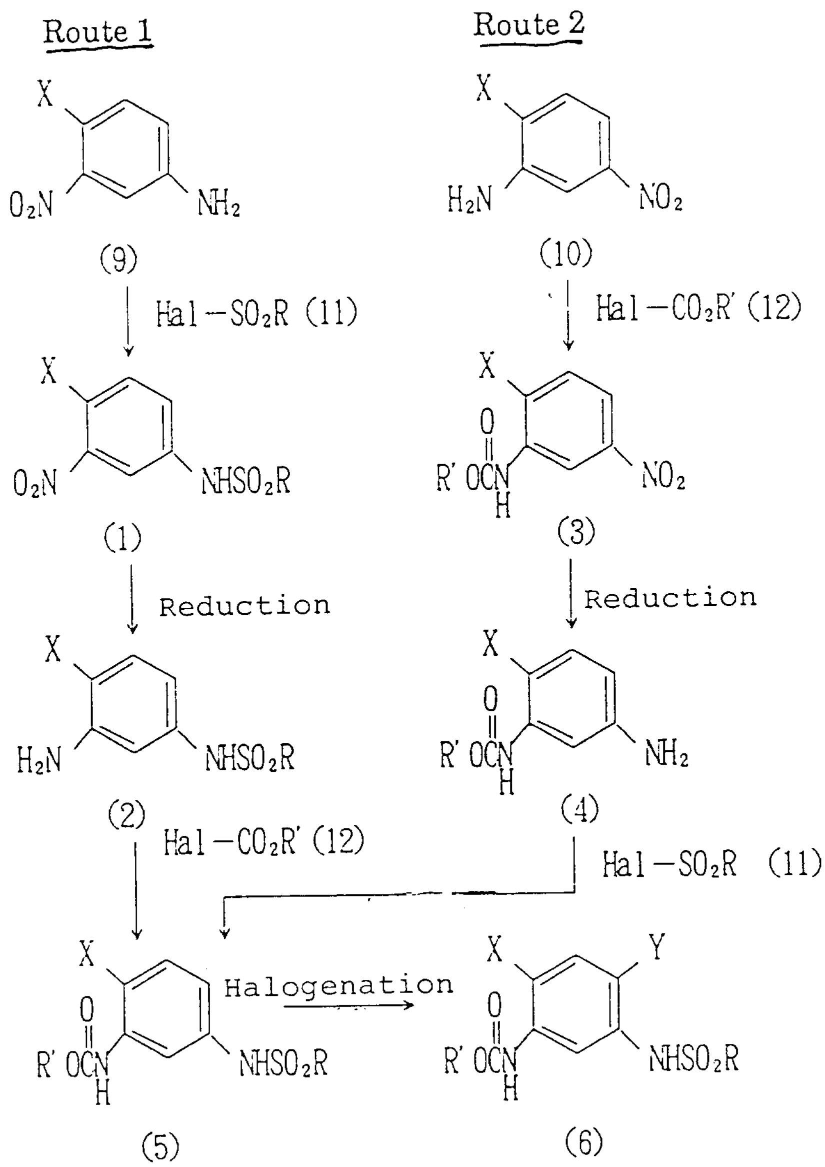


(wherein X,Y and Hal independently are halogen atoms, R is $C_1$-$C_4$ alkyl or $C_1$-$C_3$ haloalkyl and R' is $C_1$-$C_4$ alkyl or phenyl).

The present invention provides a process for producing a sulfoneamide derivative of formula (6):

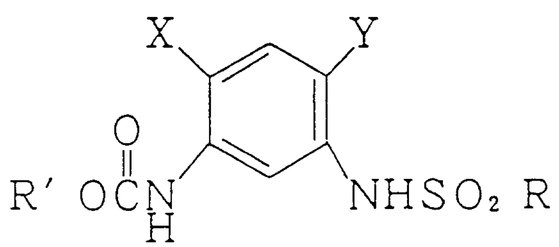

(6)

wherein X and Y independently are a halogen atom, R is $C_1$-$C_4$ alkyl or $C_1$-$C_3$ haloalkyl and R' is $C_1$-$C_4$ alkyl or phenyl, which process comprises halogenating an anilide derivative of formula (5):

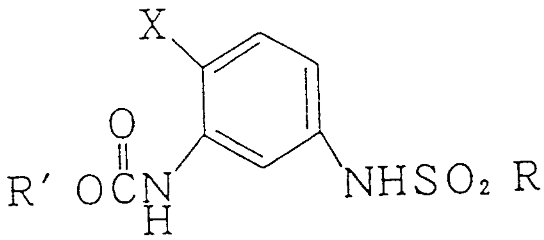

(5)

wherein X, R and R' are as defined above.

The anilide derivative of formula (5) may be produced by reacting a diamine derivative of formula (2):

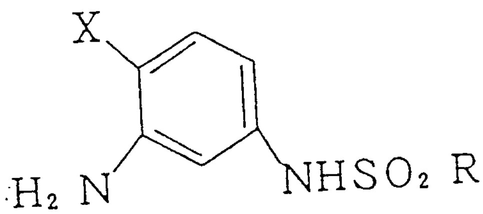

(2)

wherein X and R are as defined above, with a halogenoformate of formula (12):

Hal—$CO_2R'$ (12)

wherein Hal represents a halogen atom and R' is as defined above.

The diamine derivative of formula (2) may be produced by reducing a nitroaniline derivative of formula (1):

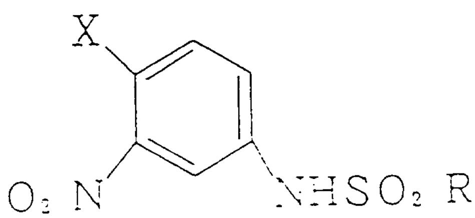

(1)

wherein X and R are as defined above.

The nitroaniline derivative of formula (1) may be produced by reacting a 4-halogeno-3-nitroaniline of formula (9):

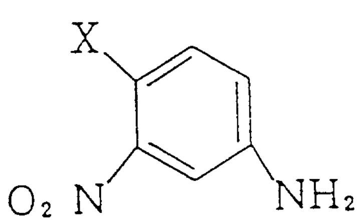

(9)

wherein X is as defined above, with an alkylsulfonyl halide of formula (11):

Hal—$SO_2R$ (11)

wherein Hal represents a halogen atom and R is as defined above.

The anilide derivative of formula (5) may also be produced by reacting an aminocarbanilide derivative of formula (4):

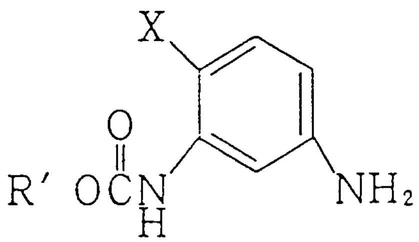

(4)

wherein X and R' are as defined above, with an alkylsulfonyl halide of formula (11) as defined above.

The aminocarbanilide derivative of formula (4) may be produced by reducing a nitrocarbanilide derivative of formula (3):

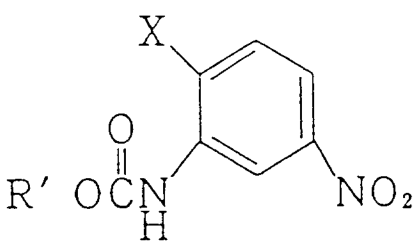

(3)

wherein R' and X are as defined above.

The nitrocarbanilide derivative of formula (3) may be produced by reacting a 2-halogeno-5-nitroaniline of formula (10):

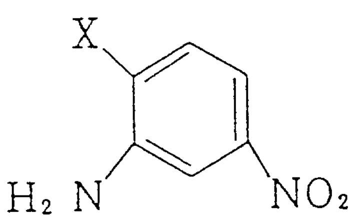

(10)

wherein X is as defined above, with a halogenoformate of formula (12) as defined above.

The present invention also provides a nitroaniline derivative of formula (1) as defined above with the provisos that when X is fluorine R is not methyl, and when X is chlorine R is not methyl, chloromethyl or trifluoromethyl.

The present invention additionally provides a diamine derivative of formula (2) as defined above with the proviso that when X is chlorine R is not methyl or trifluoromethyl.

The present invention additionally provides a process for producing a nitroaniline derivative of formula (1) as defined above with the proviso that when X is chlorine R is not methyl, chloromethyl or trifluoromethyl or a diamine derivative of formula (2) as defined above with the proviso that when X is chlorine R is not trifluoromethyl, which process comprises; either

(a) reacting a 4-halogeno-3-nitroaniline of formula (9) as defined above with an alkylsulfonyl halide of formula (11) as defined above, thereby obtaining a said nitroaniline derivative of formula (1); or

(b) reducing a nitroaniline derivative of formula (1) as defined above, thereby obtaining a said diamine derivative of formula (2).

The present invention further provides a nitrocarbanilide derivative of formula (3) as defined above with the proviso that when X is fluorine or chlorine R' is not methyl, when X is fluorine R' is not phenyl, and when X is chlorine R' is not ethyl; an aminocarbanilide derivative of formula (4) as defined above with the provisos that when X is fluorine or chlorine R' is not methyl, when X is fluorine R' is not phenyl, and when X is chlorine R' is not ethyl;

an anilide derivative of formula (5) as defined above with the proviso that when X is chlorine and R is trifluoromethyl R' is not ethyl; and a sulfonamide derivative of formula (6) as defined above.

The present invention further provides a process for producing a nitrocarbanilide derivative of formula (3) as defined above,
an aminocarbanilide derivative of formula (4) as defined above, an anilide derivative of formula (5) as defined above with the proviso that when X is chlorine and R is trifluoromethyl R' is not ethyl, or
a sulfonamide derivative of formula (6) as defined above, which process comprises;

(c) reacting a 2-halogeno-5-nitroaniline of formula (10) as defined above with a halogenoformate of formula (12) as defined above, thereby obtaining said nitrocarbanilide derivative of formula (3);
(d) reducing a nitrocarbanilide derivative of formula (3) as defined above, thereby obtaining a said aminocarbanilide derivative of formula (4);
(e) reacting an aminocarbanilide derivative of formula (4) as defined above with an alkylsulfonyl halide of formula (11) as defined above, thereby obtaining said anilide derivative of formula (5); or
(f) halogenating an anilide derivative of formula (5) as defined above, thereby obtaining a said sulfonamide derivative of formula (6).

The present invention yet further provides a process for producing a 6-haloalkyl-3-phenyluracil derivative of formula (8)

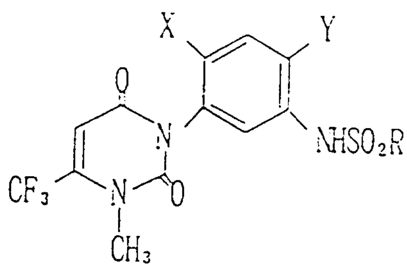

(8)

wherein X, Y and R are as defined above, which process comprises either;
(a) reacting a sulfonamide derivative of formula (6) as defined above with a compound of formula (14):

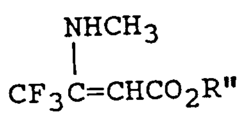

(14)

wherein R'' is $C_1$-$C_6$ alkyl, phenyl or benzyl,
or (b) reacting a sulfonamide derivative of formula (6) as defined above with a compound of formula (13):

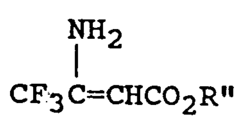

(13)

wherein R'' is as defined above, to produce an intermediate compound of formula (7):

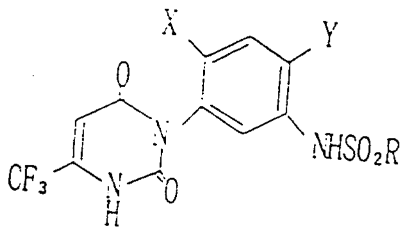

(7)

wherein X, Y and R are as defined above, and methylating said compound of formula (7). The above process may further comprise formulating the compound of formula (8) thus obtained with a herbicidally acceptable carrier or diluent.

In the above-described scheme, route 1 shows a process of producing a compound (5) with a high yield by subjecting a nitroaniline derivative (9) to sulfoneamidation, reducing the thus-obtained sulfoneamide derivative and carbamating the reduced product. Route 2 shows a process of producing a compound (5) with a high yield by carbamating a nitroaniline derivative (10), reducing the carbamated derivative and subjecting the reduced product to sulfoneamidation, and a process of producing a compound (6) with a high yield by halogenating the thus-obtained compound (5) positionally selectively.

These steps may be carried out either sequentially or continuously by appropriately selecting the conditions. A compound (8) can be manufactured from the thus-obtained compound (6) [described in JP-A-2-400475 (1990) and JP-A-3-314391 (1991)] by the following routes:

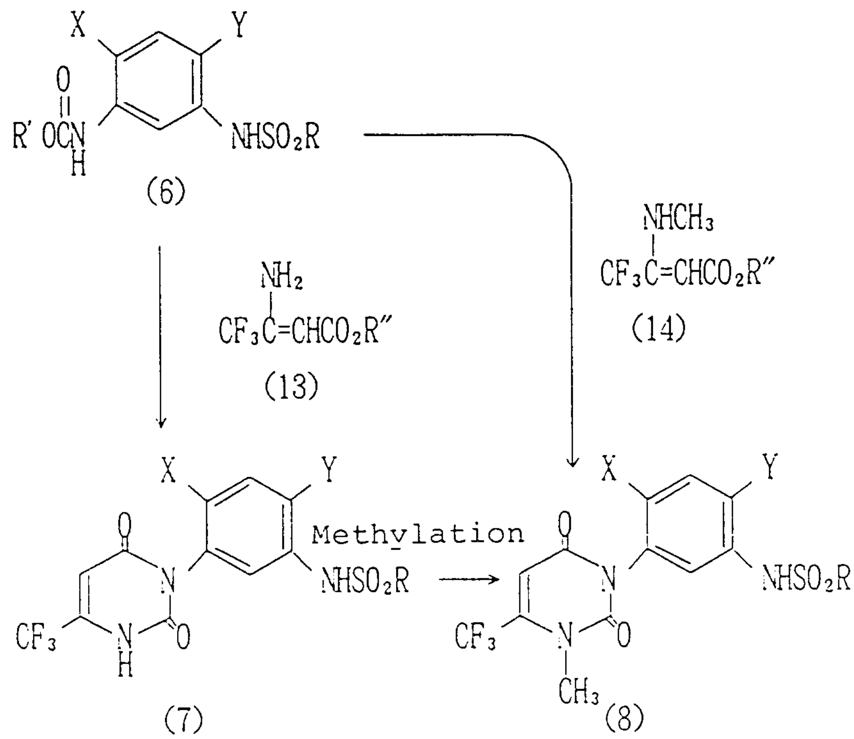

(6)
(13)
(14)
(7)
(8)

(wherein X, Y, R and R' are as defined above, and R" is $C_1$-$C_6$ alkyl, phenyl or benzyl).

The steps in the scheme will now be explained in more detail:

(A): Production of compound (1) from compound (9)

Ordinarily, 0.5 to 3.0 mol of compound (11), preferably 0.8 to 1.5 mol, based on compound (9) is used. 0.5 to 10 mol of a base, preferably 0.8 to 5.0 mol, based on compound (9) is used. The base may be used as a solvent.

Examples of the base are nitrogen-containing organic bases such as pyridine, triethylamine, N,N-dimethylaniline, N,N-diethylaniline, 4-(N,N-dimethylamino)pyridine and 1,4-diazabicyclo[2.2.2]octane, and inorganic bases such as sodium hydroxide, potassium hydroxide, sodium carbonate, potassium carbonate and sodium hydrogencarbonate.

A solvent is ordinarily necessary for the reaction. Examples of the solvent are aliphatic hydrocarbons such as hexane, heptane, ligroin and petroleum ether; aromatic hydrocarbons such as benzene, toluene, xylene and chlorobenzene; halogenated hydrocarbons such as chloroform and methylene chloride; ethers such as diethyl ether, dioxane and tetrahydrofuran; ketones such as acetone and methyl ethyl ketone; nitriles such as acetonitrile and isobutylonitrile; tertiary amines such as pyridine and N,N-diethylaniline; acid amides such as N,N-dimethylacetamide, N,N-dimethylformamide and N-methylpyrrolidone; sulfur-containing compounds such as dimethylsulfoxide and sulfolane; and mixtures thereof. Among these, the aromatic hydrocarbons, halogenated hydrocarbons, tertiary amines and mixtures thereof are preferable.

The reaction temperature is ordinarily -10 to 180 °C, preferably 0 °C to the reflux temperature.

The reaction time is ordinarily 1 to 72 hours, preferably 2 to 48 hours.

(B): Production of compound (2) from compound (1)

For reduction, a reagent reduction and catalytic hydrogenation may be adopted.

(Reagent reduction)

Ordinarily, 1 to 20 mol of a reducing agent, preferably 2 to 10 mol, based on compound (1), is used.

Examples of the reducing agent are metals and metal salts such as zinc, aluminium, tin, stannous chloride and iron.

The reaction ordinarily requires a solvent. Examples of the solvent are organic acids such as formic acid and acetic acid; inorganic acids such as hydrochloric acid, hydrobromic acid and sulfuric acid; esters such as ethyl acetate and butyl acetate; ketones such as acetone and methyl ethyl ketone; ethers such as tetrahydrofuran and dioxane; alcohols such as methanol and ethanol; water; and mixtures thereof.

The reaction temperature is ordinarily 0 to 150 °C, preferably 40 °C to the reflux temperature.

The reaction time is ordinarily 15 minutes to 24 hours, preferably 0.5 to 8 hours.

(Catalytic hydrogenation)

Ordinarily, 2.8 to 3.2 mol of hydrogen, preferably 2.9 to 3.1 mol, based on compound (1), is used. 0.001 to 10 wt% of a catalyst, preferably 0.005 to 5 wt%, based on compound (1), is used.

Examples of the catalyst are platinum catalysts such as platinum oxide (IV), platinum black, platinum carbon powder, and platinum carbon sulfide powder; palladium catalysts such as palladium carbon powder, palladium alumina powder, palladium black and palladium oxide; osmium catalysts such as osmium carbon powder; rhenium catalysts such as rhenium carbon powder; and nickel catalysts such as Raney nickel.

It is possible to add an inorganic acid such as sulfuric acid, hydrochloric acid, phosphoric acid and perchloric acid, and a base such as pyridine and triethylamine in order to accelerate the reaction.

The reaction ordinarily requires a solvent. Examples of the solvent are organic acids such as formic acid and acetic acid; esters such as ethyl acetate and butyl acetate; ethers such as diethyl ether, tetrahydrofuran and dioxane; alcohols such as methanol and ethanol; aliphatic hydrocarbons such as cyclohexane and heptane; aromatic hydrocarbons such as benzene and toluene; acid amides such as N,N-dimethylformamide; water; and mixtures thereof. Among these, the organic acides, ethers, alcohols, aliphatic hydrocarbons, water and mixtures thereof are preferable.

The reaction temperature is ordinarily 0 to 200 °C, preferably 25 to 150 °C.

The reaction time is ordinarily 0.5 to 24 hours, preferably 1 to 12 hours.

The reaction pressure is ordinarily 0 to 200 atm, preferably 0 to 100 atm (Gauge).

(C) Production of compound (5) from compound (2)

Ordinarily, 0.5 to 2.0 mol of compound (12), preferably 0.8 to 1.2 mol, based on compound (2), is used.

Ordinarily, 0.5 to 2.0 mol of a base, preferably 0.8 to 1.5 mol, based on compound (2), is used. The base may be used as a solvent.

Examples of the base are nitrogen-containing organic bases such as pyridine, triethylamine, N,N-dimethylaniline, N,N-diethylaniline, 4-(N,N-dimethylamino)pyridine and 1,4-diazabicyclo[2.2.2]octane and inorganic bases such as sodium hydroxide, potassium hydroxide, sodium carbonate, potassium carbonate and sodium hydrogencarbonate.

A solvent is ordinarily necessary for the reaction. Examples of the solvent are aliphatic hydrocarbons such as hexane, heptane, ligroin and petroleum ether; aromatic hydrocarbons such as benzene, toluene, xylene and chlorobenzene; halogenated hydrocarbons such as chloroform and methylene chloride; ethers such as diethyl ether, dioxane and tetrahydrofuran; ketones such as acetone and methyl ethyl ketone; nitriles such as acetonitrile and isobutylonitrile; tertiary amines such as pyridine and N,N-diethylaniline; acid amides such as N,N-dimethylacetamide, N,N-dimethylformamide and N-methylpyrrolidone; sulfur-containing compounds such as dimethylsulfoxide and sulfolane; and mixtures thereof. Among these, the aromatic hydrocarbons, halogenated hydrocarbons, ketones, nitriles, tertiary amines and mixtures thereof are preferable.

The reaction temperatrure is ordinarily -10 °C to the reflux temperature.

The reaction time is ordinarily 0.5 to 72 hours, preferably 1 to 12 hours.

(D) Production of compound (3) from compound (10)

Ordinarily 0.5 to 2.0 mol of compound (12), preferably 0.8 to 1.5 mol, based on compound (10), is used.

Ordinarily, 0.5 to 2.0 mol of a base, preferably 0.8 to 1.6 mol, based on compound (10), is used. The base may be used as a solvent.

Examples of the base are nitrogen-containing organic bases such as pyridine, triethylamine, N,N-dimethylaniline, N,N-diethylaniline, 4-(N,N-dimethylamino)pyridine and 1,4-diazabicyclo[2.2.2]octane and inorganic bases such as sodium hydroxide, potassium hydroxide, sodium carbonate, potassium carbonate and sodium hydrogencarbonate.

A solvent is ordinarily necessary for the reaction. Examples of the solvent are aliphatic hydrocarbons such as hexane, heptane, ligroin and petroleum ether; aromatic hydrocarbons such as benzene, toluene, xylene and chlorobenzene; halogenated hydrocarbons such as chloroform and methylene chloride; ethers such as diethyl ether, dioxane and tetrahydrofuran; ketones such as acetone and methyl ethyl ketone; nitriles such as acetonitrile and isobutylonitrile; tertiary amines such as pyridine and N,N-diethylaniline; acid amides such as N,N-dimethylacetamide, N,N-dimethylformamide and N-methylpyrrolidone; sulfur-containing compounds such as dimethylsulfoxide and sulfolane; and mixtures thereof. Among these, the aromatic hydrocarbons, halogenated hydrocarbons, ketones, nitriles, tertiary amines and mixtures thereof are preferable.

The reaction temperature is ordinarily -10 °C to the reflux temperature.

The reaction time is ordinarily 0.5 to 72 hours, preferably 1 to 48 hours.

(E) Production of compound (4) from compound (3)

The reaction is carried out under the same conditions as those in (B): Production of compound (2) from compound (1).

(F) Production of compound (5) from compound (4)

Ordinarily, 0.5 to 3.0 mol of compound (11), preferably 0.8 to 1.5 mol, based on compound (4), is used.

Ordinarily, 0.5 to 10 mol of a base, preferably 0.8 to 5.0 mol, based on compound (4), is used. The base may be used as a solvent.

Examples of the base are nitrogen-containing organic bases such as pyridine, triethylamine, N,N-dimethylaniline, N,N-diethylaniline, 4-(N,N-dimethylamino)pyridine and 1,4-diazabicyclo[2.2.2]octane, and inorganic bases such as sodium hydroxide, potassium hydroxide, sodium carbonate, potassium carbonate and sodium hydrogencarbonate.

A solvent is ordinarily necessary for the reaction. Examples of the solvent are aliphatic hydrocarbons such as hexane, heptane, ligroin and petroleum ether; aromatic hydrocarbons such as benzene, toluene, xylene and chlorobenzene; halogenated hydrocarbons such as chloroform and methylene chloride; ethers such as diethyl ether, dioxane and tetrahydrofuran; ketones such as acetone and methyl ethyl ketone; nitriles such as acetonitrile and isobutylonitrile; tertiary amines such as pyridine and N,N-diethylaniline; acid amides such as N,N-dimethylacetamide, N,N-dimethylformamide and N-methylpyrrolidone; sulfur-containing compounds such as dimethylsulfoxide and sulfolane; and mixtures thereof. Among these, the aromatic hydrocarbons, halogenated hydrocarbons, tertiary amines and mixture thereofs are preferable.

The reaction temperature is ordinarily -10 to 180 °C, preferably 0 °C to the reflux temperature.

The reaction time is ordinarily 1 to 72 hours, preferably 2 to 48 hours.

(G): Production of compound (6) from compound (5)

Ordinarily, 0.5 to 5.0 mol of a halogenating agent, preferably 0.8 to 1.5 mol, based on compound (5), is used.

Examples of the halogenating agent are halogens such as chlorine and bromine, hydrogen chloride, sulfuryl chloride, N-chlorosuccineimide, N-bromosuccineimide, N-chloroisocyanuric acid, cuprous bromide and antimony pentachloride.

A solvent is ordinarily necessary for the reaction. Examples of the solvent are organic acids such as formic acid and acetic acid; aliphatic hydrocarbons such as hexane, heptane, ligroin and petroleum ether; aromatic hydrocarbons such as benzene, toluene, xylene and chlorobenzene; halogenated hydrocarbons such as chloroform and methylene chloride; ethers such as diethyl ether, dioxane and tetrahydrofuran; sulfolane; water; and mixtures thereof. Among these, the organic acids, ethers, water and mixtures thereof

are preferable.

The reaction temperature is ordinarily 0 to 200 °C, preferably 10 to 130°C.

The reaction time is ordinarily 0.5 to 24 hours, preferably 1 to 12 hours.

The thus-obtained sulfoneamide derivative (6) is used as an intermediate for producing a 6-haloalkyl-3-phenyluracil derivative, which is useful as a herbicide, as is seen from the later-described Reference Examples.

The diamine derivative (2), the nitrocarbanilide derivative (3), the aminocarbanilide derivative (4) and the anilide derivative (5) are also useful intermediates for producing the sulfoneamide derivative (6).

[Examples]

Intermediates and a process for producing the same according to the present invention and a process for producing a herbicide by using the intermediate of the present invention will be explained in more detail with reference to the following Examples.

Example 1

Synthesis of N-(4-fluoro-3-nitrophenyl) ethanesulfonamide

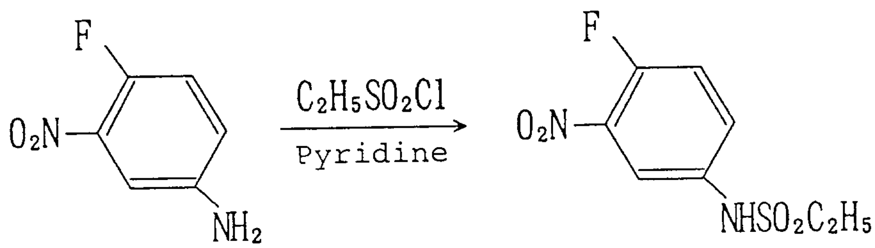


20.2 g (0.129 mol) of 4-fluoro-3-nitroaniline was dissolved in 120 m*l* of pyridine, and 17.8 g (0.136 mol) of ethanesulfonyl chloride was added dropwise to the solution at a temperature of not higher than 5 °C. Thereafter the temperature was raised to room temperature and the reaction was continued for one night. After the pyridine was distilled off, the thus-obtained product was dissolved in ethyl acetate and washed with diluted hydrochloric acid twice, then with water, and with saturated saline solution. The solution was dried over anhydrous sodium sulfate, and the ethyl acetate was distilled off to obtain the crude product (crystals). The crude product was washed with diisopropyl ether, thereby obtaining 29.7 g of the objective product (yield: 93%) in the form of brown crystals. Melting point: 133 to 136 °C

$^1$H-NMR ($d_6$- DMSO): δ

1.36 (3H, t, J = 7Hz), 3.16 (2H, q, J = 7Hz), 7.31 (1H, dd, J = 10 Hz), 7.63 (1H, ddd, J = 2, 7, 10Hz), 8.07 (1H, dd, J = 2, 7Hz), 10.66 (1H, br s)

Example 2

Synthesis of N-(3-amino-4-fluorophenyl)ethanesulfonamide

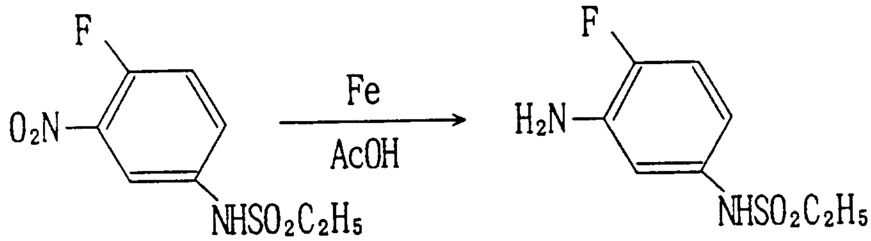


A mixture of 63.1 g (1.13 mol) of iron powder and 120 m*l* of an aqueous solution of 5% acetic acid was heated to 80 °C, and a mixed solution of 28.0 g (0.113 mol) of N-(4-fluoro-3-nitrophenyl)ethanesulfonamide, 116 m*l* of acetic acid and 116 m*l* of ethyl acetate was slowly added dropwise to the resultant mixture. After the reaction was continued for 3 hours, the insolubles were filtered out and washed with ethyl acetate. Thereafter the filtrate was distilled to remove the solvent, and the product was dissolved in ethyl acetate and washed with saturated sodium hydrogencarbonate, water and saturated saline solution. The solution was dried over anhydrous sodium sulfate, and the ethyl acetate was distilled off to obtain the crude product (crystals). The crude product was washed with diisopropyl ether, thereby obtaining 22.0 g of the objective product (yield: 89%) in the form of light yellow crystals.
Melting point: 106 to 108 °C
$^1$H-NMR ($d_6$-DMSO): δ
1.28 (3H, t, J = 7Hz), 3.05 (2H, q, J = 7Hz), 4.40 (2H, br s), 6.48 (1H, ddd, J = 2, 7, 10Hz), 6.77 (1H, dd, J = 2, 7Hz), 6.89 (1H, dd, J = 10Hz)

Example 3

Synthesis of ethyl 5-ethylsulfonylamino-2-fluorocarbanilate

(Process a)

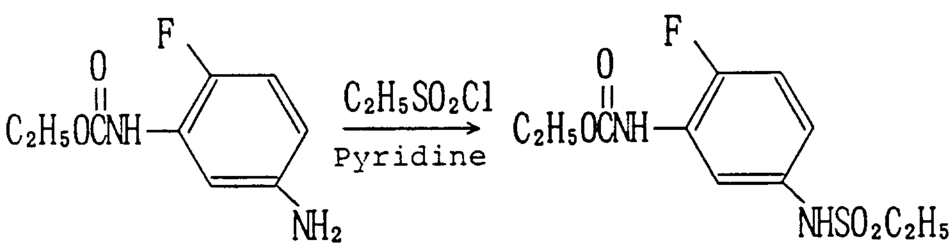


10.0 g (50.5 mmol) of ethyl 5-amino-2-fluorcarbanilate was dissolved in 60 m*l* of pyridine, and 6.82 g (53.0 mmol) of ethanesulfonyl chloride was added dropwise to the solution at a temperature of not higher than 5 °C. Thereafter the temperature was raised to room temperature and the reaction was continued for one night. After the pyridine was distilled off, the thus-obtained product was dissolved in ethyl acetate and washed with diluted hydrochloric acid twice, then with water, and with saturated saline solution. The solution was dried over anhydrous sodium sulfate, and the ethyl acetate was distilled off to obtain the crude product (crystals). The crude product was recrystallized from ethyl acetate-diisopropyl ether, thereby obtaining 9.74 g of the objective product (yield: 67%) in the form of white crystals.

(Process b)

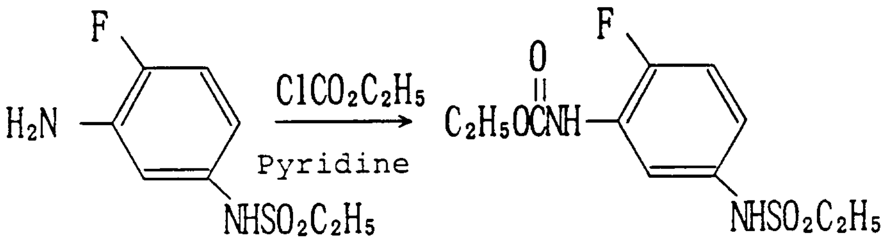


10.7 g (98.2 mmol) of ethyl chloroformate was added drop-wise to a mixture of 21.4 g (98.2 mmol) of N-(3-amino-4-fluorophenyl) ethanesulfonamide, 7.76 g (98.2 mmol) of pyridine and 214 m*l* of dichloromethane at a temperature of not higher than 5 °C. Thereafter the temperature was raised to room temperature and the reaction was continued for 5 hours. After the dichloromethane layer was separated out by adding water to the reaction mixture, the dichloromethane layer was washed with saturated saline solution and dried over anhydrous sodium sulfate. Thereafter the dichloromethane was distilled off to obtain the crude product (crystals). The crude product was washed with diisopropyl ether, thereby obtaining 28.0 g of the objective product (yield: 98%) in the form of white crystals.Melting point: 107 to 110 °C

$^1H$-NMR ($d_6$ - DMSO): δ

1.30 (6H, t, J = 7Hz), 3.06 (2H, q, J = 7Hz), 4.22 (1H, q, J = 7Hz), 7.00 (2H, br d, J = 8Hz), 7.94 (1H, br d, J = 7Hz), 8.19 (1H, br s), 10.41 (1H, br s)

Example 4

Synthesis of ethyl 2-fluoro-5-nitrocarbanilate

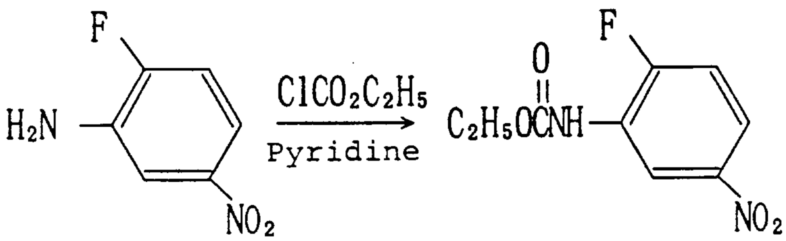


10.7 g (98.9 mmol) of ethyl chloroformate was added drop-wise to a mixture of 15.0 g (94.2 mmol) of 2-fluoro-5-nitroaniline, 8.20 g (104 mmol) of pyridine and 150 m*l* of dichloromethane at a temperature of not higher than 5 °C. Thereafter the temperature was raised to room temperature and the reaction was continued for one night. After the dichloromethane layer was separated out by adding water to the reaction mixture, the dichloromethane layer was washed with saturated saline solution and dried over anhydrous sodium sulfate. Thereafter the dichloromethane was distilled off to obtain the crude product (crystals). The crude product was washed with hexane, thereby obtaining 21.1 g of the objective product (yield: 98%) in the form of ocher crystals.

Melting point: 90 to 92 °C

$^1H$-NMR ($CDCl_3$): δ

1.34 (3H, t, J = 7Hz), 4.30 (2H, q, J = 7Hz), 7.22 (1H, br, s), 7.22 (1H, t, J = 9Hz), 7.91 (1H, ddd, J = 3, 6, 9Hz), 9.03 (1H, dd, J = 3, 6Hz)

Example 5

Synthesis of ethyl 5-amino-2-fluorocarbanilate

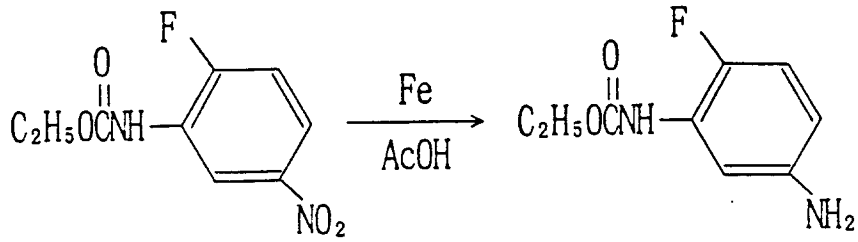


A mixture of 39.4 g (706 mmol) of iron powder and 75 m*l* of an aqueous solution of 5% acetic acid was heated to 80 °C, and a mixed solution of 16.1 g (70.6 mmol) of ethyl 2-fluoro-5-nitrocarbanilate, 72 m*l* of acetic acid and 72 m*l* of ethyl acetate was slowly added dropwise to the mixture. After the reaction was continued for 3 hours, the insolubles were filtered out and washed with ethyl acetate. Thereafter the filtrate was distilled to remove the solvent, and the resultant product was dissolved in ethyl acetate and washed with saturated sodium hydrogen-carbonate, water and saturated saline solution. The solution was dried over anhydrous sodium sulfate, and the ethyl acetate was distilled off to obtain the crude product (crystals). The crude product was washed with diisopropyl ether, thereby obtaining 12.8 g of the objective product (yield: 91%) in the form of light yellow crystals.
Melting point: 88 to 89 °C
$^1$H-NMR ($CDCl_3$): $\delta$
1.24 (3H, t, J = 7Hz), 3.78 (2H, br s), 4.19 (2H, q, J = 7Hz), 6.23 (1H, ddd, J = 2, 7, 10Hz), 6.80 (1H, dd, J = 10 Hz), 7.23 (1H, br s), 7.44 (1H, dd, J = 2, 7Hz)

Example 6

Synthesis of ethyl 4-chloro-5-ethylsulfonylamino-2-fluorocarbanilate

(Process a)

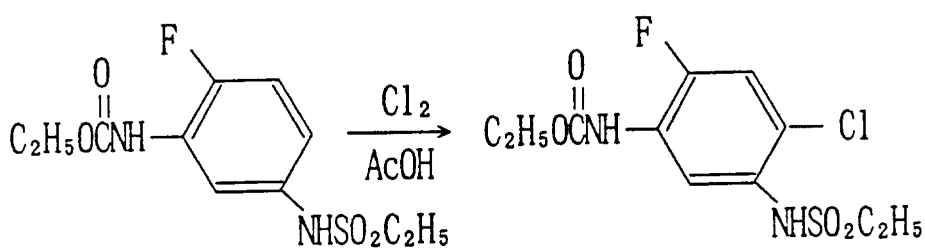


1.00 g (3.45 mmol) of ethyl 5-ethylsulfonylamino-2-fluorocarbanilate was dissolved in 10 m*l* of acetic acid, and chlorine gas was added to the resultant solution at a temperature of not higher than 25 °C until the starting material disappeared. After the reaction was completed, the surplus chlorine gas was removed by blowing nitrogen gas thereinto, and diisopropyl ether was added. The precipitated crystals were filtered out to obtain 0.76 g of the objective product (yield: 68%) in the form of white crystals.

(Process b)

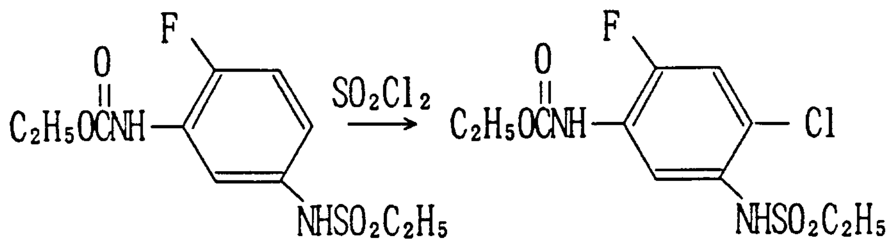


0.50 g (1.72 mmol) of ethyl 5-ethylsulfonylamino-2-fluorocarbanilate was added to 4 m*l* of sulfuryl chloride. The sulfuryl chloride was distilled off after 10 minutes, and the resultant product was dissolved in ethyl acetate. The solution was washed with water and a saturated saline solution, and dried over anhydrous sodium sulfate. The ethyl acetate was distilled off to obtain the crude product. The crude product was washed with diisopropyl ether, thereby obtaining 0.21 g of the objective product (yield: 38%) in the form of white crystals.

(Process c)

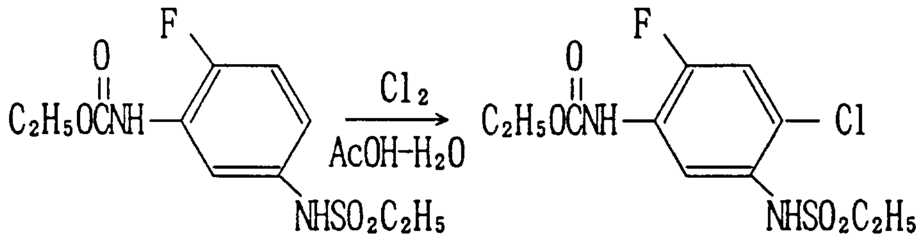


0.50 g (1.72 mmol) of ethyl 5-ethylsulfonylamino-2-fluorocarbanilate was dissolved in a mixed solution of 12 m*l* of acetic acid and 1 m*l* of water, and chlorine gas was added to the resultant solution at a temperature of not higher than 25 °C until the starting material disappeared. After the reaction was completed, the surplus chlorine gas was removed by blowing nitrogen gas thereinto, and water was added. The precipitated crystals were filtered out and dried to obtain 0.49 g of the objective product (yield: 88%) in the form of white crystals.
Melting point: 162 to 164 °C
$^1$H-NMR ($d_6$-DMSO): δ
1.28 (3H, t, J = 7Hz), 1.32 (3H, t, J = 7Hz), 3.08 (2H, q, J = 7Hz), 4.12 (2H, q, J = 7Hz), 7.13 (1H, d, J = 9Hz), 7.88 (1H, d, J = 7Hz), 8.88 (1H, br s), 8.94 (1H, br s)

Example 7

Synthesis of N-(3-amino-4-fluorophenyl)ethanesulfonamide

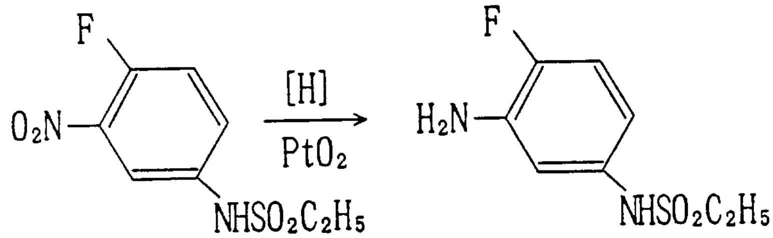


50.4 mg (5 wt%) of platinum oxide (Adams catalyst) was added to a mixture of 1.00 g (4.03 mmol) of N-(4-fluoro-3-nitrophenyl)ethanesulfoneamide and 40 m*l* of ethanol, and the resultant mixture was stirred until 285 m*l* of hydrogen was absorbed. The reaction mixture was filtered through a glass filter to remove the catalyst, and the solvent was then distilled off under a reduced pressure. Thus, 850 mg of the objective product (yield: 97%) was obtained in the form of light brown crystals.

Example 8

Synthesis of methyl 5-ethylsulfonylamino-2-fluorocarbanilate

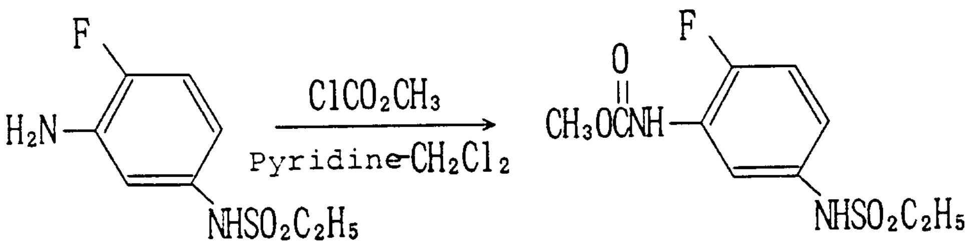


22.1 g (229 mmol) of methyl chloroformate was added drop-wise to a mixture of 50.0 g (229 mmol) of N-(3-amino-4-fluorophenyl)ethanesulfonamide, 18.1 g (229 mmol) of pyridine and 500 m*l* of dichloromethane at a temperature of not higher than 5 °C. Thereafter the temperature was raised to room temperature and the reaction was continued for 5 hours. After the dichloromethane layer was separated out by adding water to the resultant mixture, the dichloromethane layer was washed with saturated saline solution and dried over anhydrous sodium sulfate. Thereafter the dichloromethane was distilled off to obtain the crude product (crystals). The crude product was washed with diisopropyl ether, thereby obtaining 61.0 g of the objective product (yield: 96%) in the form of light brown crystals.

Example 9

Synthesis of ethyl 5-amino-2-fluorocarbanilate

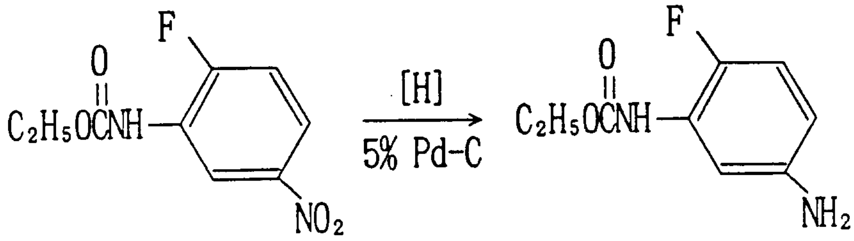


100.3 mg (10 wt%) of 5% palladium carbon (containing water) was added to a mixture of 1.00 g (4.39 mmol) of ethyl 2-fluoro-5-nitrocarbanilate and 20 m*l* of ethanol, and the resultant mixture was stirred until 309 m*l* of hydrogen was absorbed. The reaction mixture was filtered to remove the catalyst, and the solvent was then distilled off under a reduced pressure. Thus, 700 mg of the objective product (yield: 81%) was obtained in the form of light brown crystals.

Example 10

Synthesis of ethyl 5-methylsulfonylamino-2-fluorocarbanilate

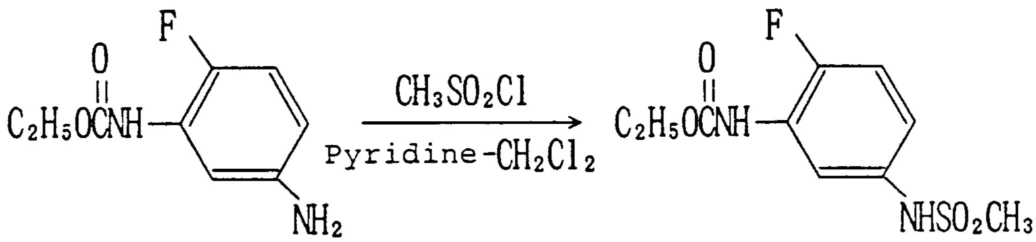


61.1 g (533 mmol) of methanesulfonyl chloride was added dropwise to a mixture of 96.0 g (485 mmol) of ethyl 5-amino-2-fluorocarbanilate, 46.0 g (582 mmol) of pyridine and 700 m*l* of dichloromethane at a temperature of not higher than 5 °C. Thereafter the temperature was raised to room temperature and the reaction was continued for one night. After the solvent was distilled off, the resultant product was dissolved in ethyl acetate and washed with diluted hydrochloric acid twice, then with water, and with saturated saline solution. The resultant solution was dried over anhydrous sodium sulfate, and the ethyl acetate was distilled off to obtain the crude procut (crystals). The crude product was washed with diisopropyl ether, thereby obtaining 126 g of the objective product (yield: 94%) in the form of light brown crystals.

Example 11

synthesis of ethyl 4-chloro-5-ethylsulfonylamino-2-fluorocarbanilate

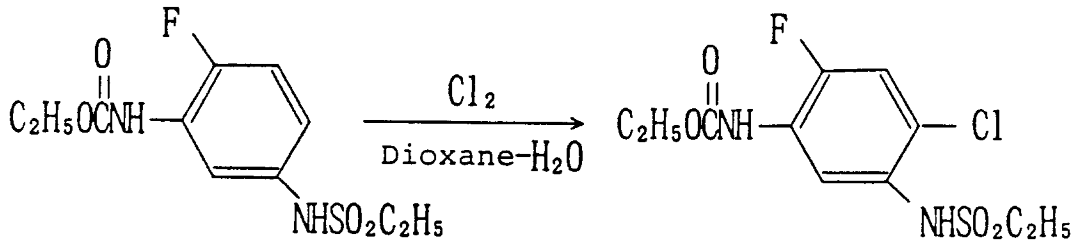


0.50 g (1.72 mmol) of ethyl 5-ethylsulfonylamino-2-fluorocarbanilate was dissolved in a mixture of 5 m*l* of dioxane and 1 m*l* of water, and chlorine gas was added to the resultant solution at a temperature of not higher than 25 °C until the starting material disappeared. After the reaction was completed, the surplus chlorine gas was removed by blowing nitrogen gas thereinto, and water was added. The precipitates crystals were filtered out and dried to obtain 0.48 g of the objective product (yield: 86%) in the form of light brown crystals.

Example 12

Synthesis of ethyl 4-chloro-5-methylsulfonylamino-2-fluorocarbanilate

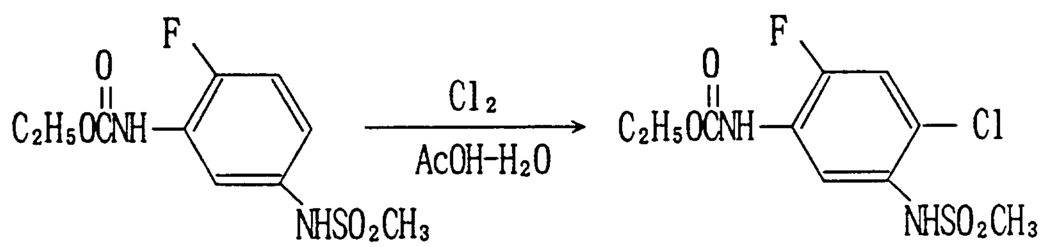


80.6 g (292 mmol) of ethyl 5-methylsulfonylamino-2-fluorocarbanilate was dissolved in a mixture of 1075 m*l* of acetic acid and 100 m*l* of water, and chlorine gas was added to the resultant solulion at a temperature of not higher than 25 °C until the starting material disappeared. After the reaction was completed, the surplus chlorine gas was removed by blowing nitrogen gas thereinto, and hexane was added. The precipitates crystals were filtered out to obtain 81.4 g of the objective product (yield: 90%) in the form of yellow crystals.

Example 13

Synthesis of 3-(4-chloro-5-ethylsulfonylamino-2-fluorophenyl)-6-trifluoromethyl-2,4(1H,3H)-pyrimidinedione

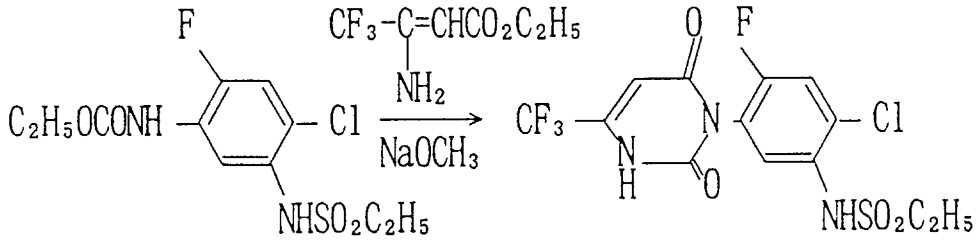


1.06 g (5.77 mmol) of ethyl 3-amino-4,4,4-trifluorocrotonate was dissolved in 6.2 m*l* of N,N-dimethylformamide and 0.82 g (14.4 mmol) of sodium methoxide was added thereto. The reaction mixture was cooled to not higher than 5 °C after 10 minutes, and 1.56 g (4.81 mmol) of ethyl 4-chloro-5-ethylsulfonylamino-2-fluorocarbanilate was added thereto. The resultant mixture was heated to 110 °C and reacted for 4 hours. After the reaction was completed, N,N-dimethylformamide was distilled off and the resultant product was dissolved in water. The thus-obtained solution was washed with diethyl ether 3 times and thereafter concentrated hydrochloric acid was added thereto to pH 2. The precipitated crystals were filtered out, washed with water and dried to obtain 1.54 g of the objective product (yield: 77%) in the form of light yellow crystals.
Melting point: 190 to 192 °C
$^1$H-NMR ($d_6$-DMSO): δ
1.36 (3H, t, J = 7Hz), 3.12 (2H, q, J = 7Hz), 6.19 (1H, s), 7.44 (1H, d, J = 9Hz), 7.58 (1H, d, J = 7Hz), 8.86 (1H, br s), 9.20 (1H, br s)

Reference Example 1

Synthesis of 3-(4-chloro-5-ethylsulfonylamino-2-fluorophenyl)-1-methyl-6-trifluoromethyl-2,4(1H,3H)-pyrimidinedione

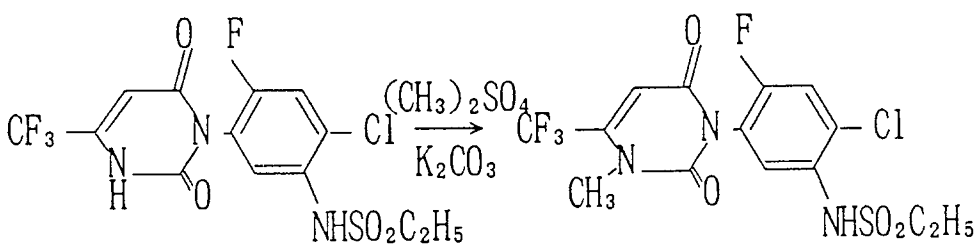


1.00 g (2.41 mmol) of 3-(4-chloro-5-ethylsulfonylamino-2-fluorophenyl)-6-trifluoromethyl-2,4 (1H, 3H)-pyrimidinedione was dissolved in 10 m*l* of acetone, and 0.17 g (1.20 mmol) of anhydrous potassium carbonate and 0.23 m*l* (2.41 mmol) of dimethyl sulfate were added thereto and the reaction was continued for 1.5 hours. After acetone was distilled off, the reaction product was dissolved in ethyl acetate, washed with water and saturated saline solution, and dried over anhydrous sodium sulfate. Thereafter the ethyl acetate was distilled off to obtain a crude product. The crude product was recrystallized from chloroformdiethyl ether, thereby obtaining 0.61 g of the objective product (yield: 59 %) in the form of light yellow crystals.
Melting point: 176 to 177 °C
$^1$H-NMR ($d_6$-DMSO): δ
1.32 (3H, t, J = 7Hz), 3.06 (2H, q, J = 7Hz), 3.43 (3H, s), 6.23 (1H, s), 7.29 (1H, d, J = 9Hz), 7.41 (1H, d, J = 7Hz), 9.11 (1H, br s)

Reference Example 2

Test of herbicidal effect of soil treatment

50 parts of 3-(4-chloro-5-ethylsulfonylamino-2-fluorophenyl)-1-methyl-6-trifluoromethyl-2,4-(1H,3H)-pyrimidinedione, 47 parts of Zeeklite (Kaolin clay, produced by Zeeklite Mining Industries Co., Ltd.), 2 parts of Sorpol 5039 (anionic surfactant, produced by Toho Chemical Ind. Co., Ltd.) and 1 part of Carplex (white carbon, produced by Shionogi Co., Ltd.) were uniformly mixed and pulverized to produce a wettable powder.

Sterilized diluvial soil was placed into a plastic box [15 cm(long) x 22 cm(wide) x 6 cm(deep)] , and sowed at random with the seeds of Echinochloa crus-qalli, Digitaria, adscendens, Cyperus microiria, Solanum nigrum, Galinsoga ciliata, Rorippa indica, rice, corn, wheat, soybean and cotton. The diluvial soil was then covered with soil to a depth of about 1 cm, and the wettable powder diluted with water was uniformly sprayed onto the surface of the soil at a predetermined rate. The herbicidal effect on the respective grasses was examined after 3 weeks in accordance with the following evaluation criteria:

Standard ratings:

5: Growth control rate of more than 90% (almost completely withered)
4: Growth control rate of from 70 to 90%
3: Growth control rate of from 40 to 70%
2: Growth control rate of from 20 to 40%
1: Growth control rate of from 5 to 20%
0: Growth control rate of less than 5% (almost non-effective)

The above growth control rates were calculated by the following equation:

$$\text{Growth control rate (\%)} = (1 - T/N) \times 100$$

where

T: weight of the weed growth above the soil surface of the treated area,
N: weight of the weed grown above the soil surface of the non-treated area.

In Table 1, the respective symbols represent the following grasses.

N (Echinochloa crus-qalli), M (Digitaria adscendens), K (Cyperus microiria), H (Solanum nigrum), D (Galinsoga ciliata), I (Rorippa indica), R (rice), T (corn), W (wheat), S (soybean) and C (cotton)

Table 1

| Dosage (g/ha) | N | M | K | H | D | I | R | T | W | S | C |
|---|---|---|---|---|---|---|---|---|---|---|---|
| 10 | 5 | 3 | 5 | 5 | 5 | 5 | 0 | 0 | 0 | 0 | 2 |
| 20 | 5 | 4 | 5 | 5 | 5 | 5 | 1 | 0 | 0 | 0 | 4 |
| 40 | 5 | 5 | 5 | 5 | 5 | 5 | 3 | 0 | 0 | 0 | 5 |

## Claims

**Claims for the following Contracting States : GB, DE, FR, IT, NL, CH, BE, AT, SE, GR, LU, DK, MC, PT, LI**

1. A process for producing a sulfonamide derivative of formula (6):

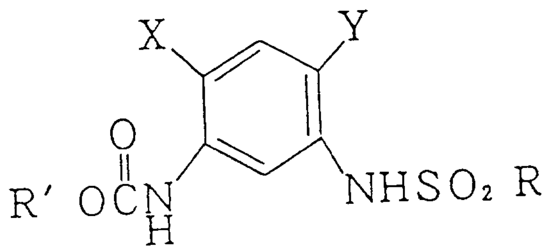

(6)

wherein X and Y independently are a halogen atom, R is $C_1$-$C_4$ alkyl or $C_1$-$C_3$ haloalkyl and R' is $C_1$-$C_4$ alkyl or phenyl, which process comprises halogenating an anilide derivative of formula (5):

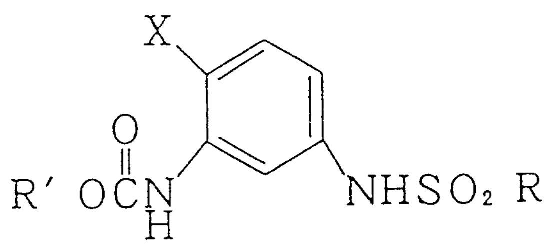

(5)

wherein X, R and R' are as defined above.

2. A process according to claim 1 in which the anilide derivative of formula (5) is produced by reacting a diamine derivative of formula (2):

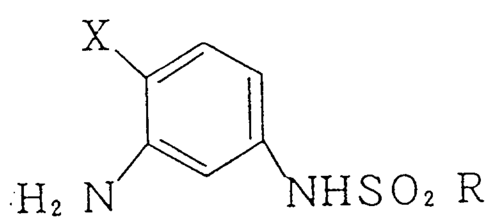

(2)

wherein X and R are as defined in claim 1, with a halogenoformate of formula (12):

Hal—$CO_2$R'     (12)

wherein Hal represents a halogen atom and R' is as defined in claim 1.

3. A process according to claim 2 in which the diamine derivative of formula (2) is produced by reducing a nitroaniline derivative of formula (1):

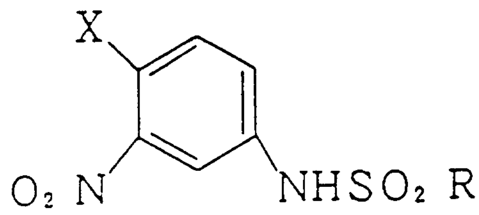

(1)

wherein X and R are as defined in claim 1.

**4.** A process according to claim 3 in which the nitroaniline derivative of formula (1) is produced by reacting a 4-halogeno-3-nitroaniline of formula (9):

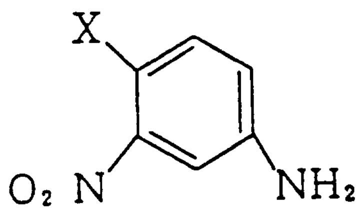

(9)

wherein X is as defined in claim 1, with an alkylsulfonyl halide of formula (11):

$Hal—SO_2R$ (11)

wherein Hal represents a halogen atom and R is as defined in claim 1.

**5.** A process according to claim 1 in which the anilide derivative of formula (5) is produced by reacting an aminocarbanilide derivative of formula (4):

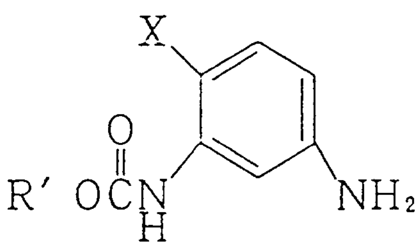

(4)

wherein X and R' are as defined in claim 1, with an alkylsulfonyl halide of formula (11) as defined in claim 4.

**6.** A process according to claim 5 in which the aminocarbanilide derivative of formula (4) is produced by reducing a nitrocarbanilide derivative of formula (3):

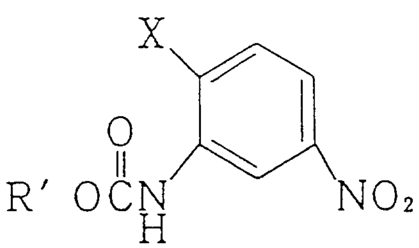

(3)

wherein R' and X are as defined in claim 1.

**7.** A process according to claim 6 in which the nitrocarbanilide derivative of formula (3) is produced by reacting a 2-halogeno-5-nitroaniline of formula (10):

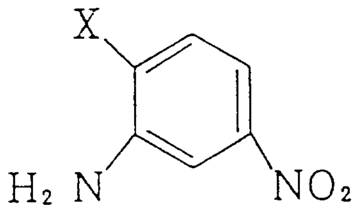

(10)

wherein X is as defined in claim 1, with a halogenoformate of formula (12) as defined in claim 2.

**8.** A nitroaniline derivative of formula (1) as defined in claim 3 with the provisos that when X is fluorine R is not methyl, and when X is chlorine R is not methyl, chloromethyl or trifluoromethyl; and a diamine derivative of formula (2) as defined in claim 2 with the proviso that when X is chlorine R is not methyl or trifluoromethyl.

**9.** A process for producing a nitroaniline derivative of formula (1) as defined in claim 3 with the proviso that when X is chlorine R is not methyl, chloromethyl or trifluoromethyl or a diamine derivative of formula (2) as defined in claim 2 with the proviso that when X is chlorine R is not trifluoromethyl, which process comprises; either

(a) reacting a 4-halogeno-3-nitroaniline of formula (9) as defined in claim 4 with an alkylsulfonyl halide of formula (11) as defined in claim 4, thereby obtaining a said nitroaniline derivative of formula (1); or

(b) reducing a nitroaniline derivative of formula (1) as defined in claim 3, thereby obtaining a said diamine derivative of formula (2).

**10.** A nitrocarbanilide derivative of formula (3) as defined in claim 6 with the provisos that when X is fluorine or chlorine R' is not methyl, when X is fluorine R' is not phenyl, and when X is chlorine R' is not ethyl;

an aminocarbanilide derivative of formula (4) as defined in claim 5 with the provisos that when X is fluorine or chlorine R' is not methyl, when X is fluorine R' is not phenyl, and when X is chlorine R' is not ethyl;

an anilide derivative of formula (5) as defined in claim 1 with the proviso that when X is chlorine and R is trifluoromethyl R' is not ethyl;

and

a sulfonamide derivative of formula (6) as defined in claim 1.

**11.** A process for producing a nitrocarbanilide derivative of formula (3) as defined in claim 6, an aminocarbanilide derivative of formula (4) as defined in claim 5,

an anilide derivative of formula (5) as defined in claim 1 with the proviso that when X is chlorine and R is trifluoromethyl R' is not ethyl, or

a sulfonamide derivative of formula (6) as defined in claim 1, which process comprises;

(c) reacting a 2-halogeno-5-nitroaniline of formula (10) as defined in claim 7 with a halogenoformate of formula (12) as defined in claim 7, thereby obtaining a said nitrocarbanilide derivative of formula (3);

(d) reducing a nitrocarbanilide derivative of formula (3) as defined in claim 6, thereby obtaining a said aminocarbanilide derivative of formula (4);

(e) reacting an aminocarbanilide derivative of formula (4) as defined in claim 5 with an alkylsulfonyl halide of formula (11) as defined in claim 4, thereby obtaining said anilide derivative of formula (5); or

(f) halogenating an anilide derivative of formula (5) as defined in claim 1, thereby obtaining a said sulfonamide derivative of formula (6).

**12.** A process for producing a 6-haloalkyl-3-phenyluracil derivative of formula (8)

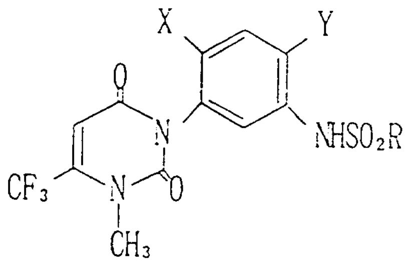

(8)

wherein X, Y and R are as defined in claim 1, which process comprises either;

(a) reacting a sulfonamide derivative of formula (6) as defined in claim 1 with a compound of formula (14):

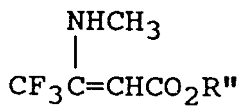

(14)

wherein R" is $C_1$-$C_6$ alkyl, phenyl or benzyl,

or (b) reacting a sulfonamide derivative of formula (6) as defined in claim 1 with a compound of formula (13):

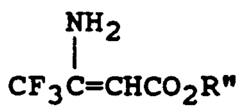

(13)

wherein R" is as defined above, to produce an intermediate compound of formula (7):

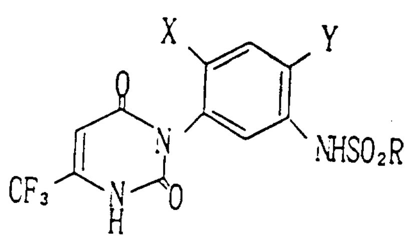

(7)

wherein X, Y and R are as defined in claim 1, and methylating said compound of formula (7).

**13.** A process according to claim 12 which further comprises formulating the compound of formula (8) thus obtained with a herbicidally acceptable carrier or diluent.

**Claims for the following Contracting State : ES**

1. A process for producing a sulfonamide derivative of formula (6):

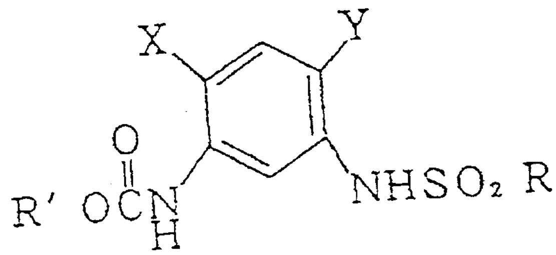

(6)

wherein X and Y independently are a halogen atom, R is $C_1$-$C_4$ alkyl or $C_1$-$C_3$ haloalkyl and R' is $C_1$-$C_4$ alkyl or phenyl, which process comprises halogenating an anilide derivative of formula (5):

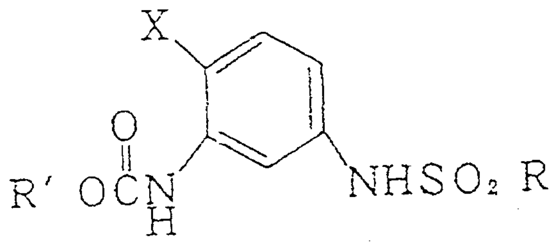

(5)

wherein X, R and R' are as defined above.

2. A process according to claim 1 in which the anilide derivative of formula (5) is produced by reacting a diamine derivative of formula (2):

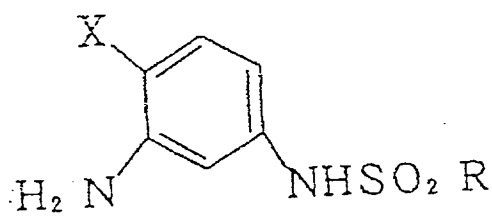

(2)

wherein X and R are as defined in claim 1, with a halogenoformate of formula (12):

$Hal—CO_2R'$ (12)

wherein Hal represents a halogen atom and R' is as defined in claim 1.

3. A process according to claim 2 in which the diamine derivative of formula (2) is produced by reducing a nitroaniline derivative of formula (1):

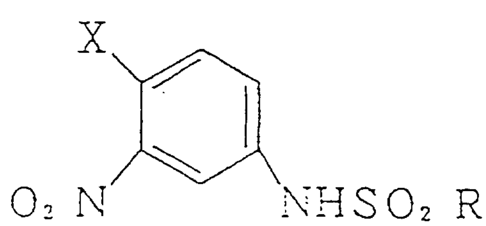

(1)

wherein X and R are as defined in claim 1.

**4.** A process according to claim 3 in which the nitroaniline derivative of formula (1) is produced by reacting a 4-halogeno-3-nitroaniline of formula (9):

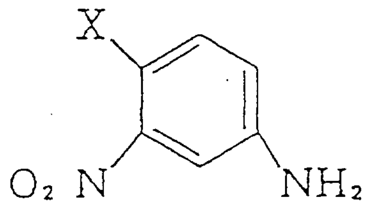

(9)

wherein X is as defined in claim 1, with an alkylsulfonyl halide of formula (11):

Hal—$SO_2$R (11)

wherein Hal represents a halogen atom and R is as defined in claim 1.

**5.** A process according to claim 1 in which the anilide derivative of formula (5) is produced by reacting an aminocarbanilide derivative of formula (4):

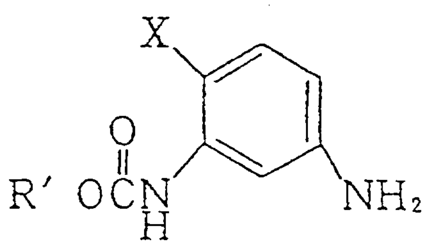

(4)

wherein X and R' are as defined in claim 1, with an alkylsulfonyl halide of formula (11) as defined in claim 4.

**6.** A process according to claim 5 in which the aminocarbanilide derivative of formula (4) is produced by reducing a nitrocarbanilide derivative of formula (3):

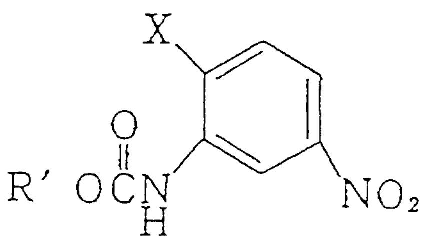

(3)

wherein R' and X are as defined in claim 1.

**7.** A process according to claim 6 in which the nitrocarbanilide derivative of formula (3) is produced by reacting a 2-halogeno-5-nitroaniline of formula (10):

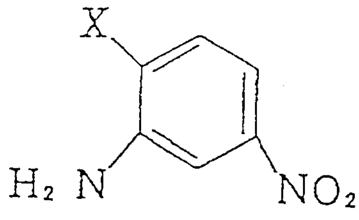

(10)

wherein X is as defined in claim 1, with a halogenoformate of formula (12) as defined in claim 2.

**8.** A process for producing a nitroaniline derivative of formula (1) as defined in claim 3 with the proviso that when X is chlorine R is not methyl, chloromethyl or trifluoromethyl or a diamine derivative of formula (2) as defined in claim 2 with the proviso that when X is chlorine R is not trifluoromethyl, which process comprises; either

(a) reacting a 4-halogeno-3-nitroaniline of formula (9) as defined in claim 4 with an alkylsulfonyl halide of formula (11) as defined in claim 4, thereby obtaining a said nitroaniline derivative of formula (1); or

(b) reducing a nitroaniline derivative of formula (1) as defined in claim 3, thereby obtaining a said diamine derivative of formula (2).

**9.** A process for producing a nitrocarbanilide derivative of formula (3) as defined in claim 6,
an aminocarbanilide derivative of formula (4) as defined in claim 5,
an anilide derivative of formula (5) as defined in claim 1 with the proviso that when X is chlorine and R is trifluoromethyl R' is not ethyl, or
a sulfonamide derivative of formula (6) as defined in claim 1, which process comprises:

(c) reacting a 2-halogeno-5-nitroaniline of formula (10) as defined in claim 7 with a halogenoformate of formula (12) as defined in claim 7, thereby obtaining a said nitrocarbanilide derivative of formula (3);

(d) reducing a nitrocarbanilide derivative of formula (3) as defined in claim 6, thereby obtaining a said aminocarbanilide derivative of formula (4);

(e) reacting an aminocaroanilide derivative of formula (4) as defined in claim 5 with an alkylsulfonyl halide of formula (11) as defined in claim 4, thereby obtaining said anilide derivative of formula (5); or

(f) halogenating an anilide derivative of formula (5) as defined in claim 1, thereby obtaining a said sulfonamide derivative of formula (6).

**10.** A process for producing a 6-haloalkyl-3-phenyluracil derivative of formula (8)

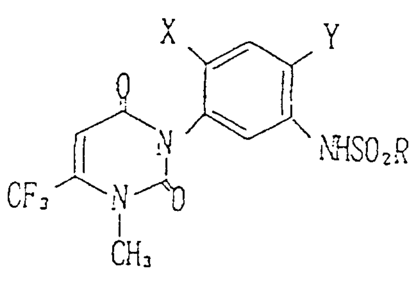

(8)

wherein X, Y and R are as defined in claim 1, which process comprises either;

(a) reacting a sulfonamide derivative of formula (6) as defined in claim 1 with a compound of formula (14):

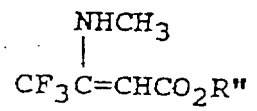

(14)

Wherein R" is $C_1$-$C_6$ alkyl, phenyl or benzyl,
or (b) reacting a sulfonamide derivative of formula (6) as defined in claim 1 with a compound of formula (13):

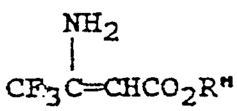

(13)

wherein R" is as defined above, to produce an intermediate compound of formula (7):

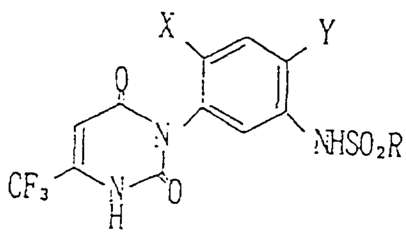

(7)

wherein X, Y and R are as defined in claim 1, and methylating said compound of formula (7).

**11.** A process according to claim 10 which further comprises formulating the compound of formula (8) thus obtained with a herbicidally acceptable carrier or diluent.

**Patentansprüche**

**Patentansprüche für folgende Vertragsstaaten : GB, DE, FR, IT, NL, CH, BE, AT, SE, GR, LU, DK, MC, PT, LI**

**1.** Verfahren zur Herstellung eines Sulfonamidderivats der Formel (6):

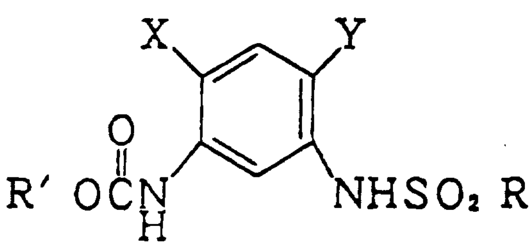

(6),

worin X und Y jeweils unabhängig ein Halogenatom sind, R $C_1$-$C_4$-Alkyl oder $C_1$-$C_3$-Halogenalkyl ist, und R' $C_1$-$C_4$-Alkyl oder Phenyl ist, wobei das Verfahren die Halogenierung eines Anilidderivats der Formel (5) umfaßt:

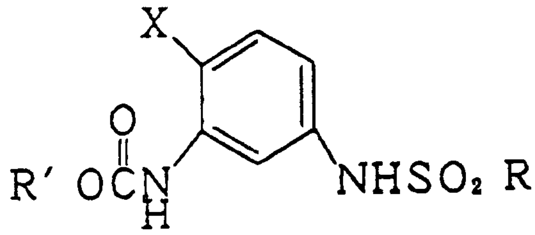

(5),

worin X, R und R' wie oben definiert sind.

**2.** Verfahren gemäß Anspruch 1, worin das Anilidderivat der Formel (5) hergestellt wird durch Umsetzung eines Diaminderivats der Formel (2):

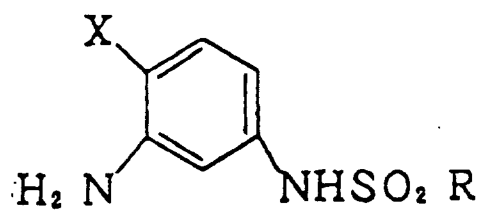

(2),

worin X und R wie in Anspruch 1 definiert sind,
mit einem Halogenformiat der Formel (12):

Hal—$CO_2$R'     (12),

worin Hal ein Halogenatom darstellt, und R' wie in Anspruch 1 definiert ist.

**3.** Verfahren gemäß Anspruch 2, worin das Diaminderivat der Formel (2) hergestellt wird durch Reduktion eines Nitroanilinderivats der Formel (1):

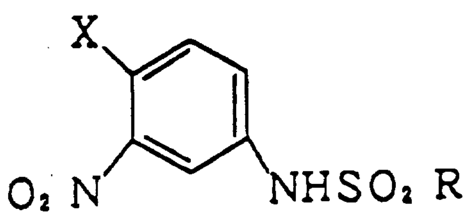

(1),

worin X und R wie in Anspruch 1 definiert sind.

**4.** Verfahren gemäß Anspruch 3, worin das Nitroanilinderivat der Formel (1) hergestellt wird durch Umsetzung eines 4-Halogen-3-nitroanilins der Formel (9):

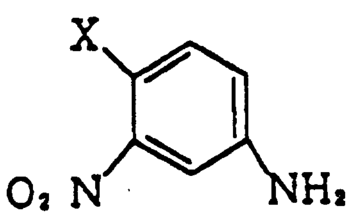

(9),

worin X wie in Anspruch 1 definiert ist,
mit einem Alkylsulfonylhalogenid der Formel (11):

Hal—$SO_2$R (11),

worin Hal ein Halogenatom darstellt und R wie in Anspruch 1 definiert ist.

**5.** Verfahren gemäß Anspruch 1, worin das Anilidderivat der Formel (5) hergestellt wird durch Umsetzung eines Aminocarbanilidderivats der Formel (4):

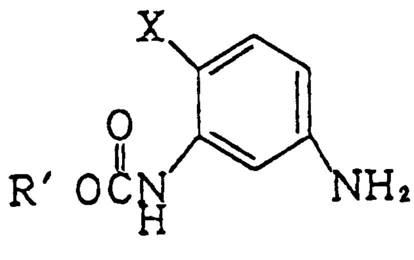

(4) ,

worin X und R' wie in Anspruch 1 definiert sind, mit einem Alkylsulfonylhalogenid der Formel (11), wie in Anspruch 4 definiert.

**6.** Verfahren gemäß Anspruch 5, worin das Aminocarbanilidderivat der Formel (4) hergestellt wird durch Reduktion eines Nitrocarbanilidderivats der Formel (3):

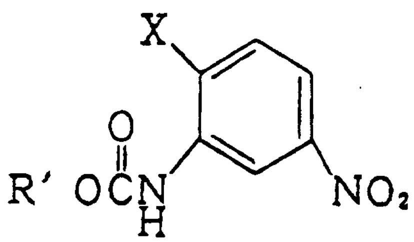

(3) ,

worin R' und X wie in Anspruch 1 definiert sind.

**7.** Verfahren gemäß Anspruch 6, worin das Nitrocarbanilidderivat der Formel (3) hergestellt wird durch Umsetzung eines 2-Halogen-5-nitroanilins der Formel (10):

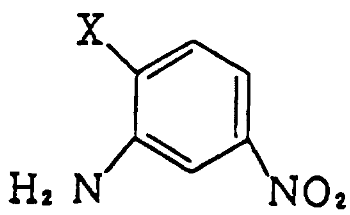

(10) ,

worin X wie in Anspruch 1 definiert ist,
mit einem Halogenformiat der Formel (12), wie in Anspruch 2 definiert.

**8.** Nitroanilinderivat der Formel (1), wie in Anspruch 3 definiert, mit der Maßgabe, daß, falls X Fluor ist, R nicht Methyl ist, und falls X Chlor ist, R weder Methyl noch Chlormethyl oder Trifluormethyl ist; und Diaminderivat der Formel (2), wie in Anspruch 2 definiert, mit der Maßgabe, daß, falls X Chlor ist, R weder Methyl noch Trifluormethyl ist.

**9.** Verfahren zur Herstellung eines Nitroanilinderivats der Formel (1), wie in Anspruch 3 definiert, mit der Maßgabe, daß, falls X Chlor ist, R nicht Methyl, Chlormethyl oder Trifluormethyl ist,

oder eines Diaminderivats der Formel (2), wie in Anspruch 2 definiert, mit der Maßgabe, daß, falls X Chlor ist, R nicht Trifluormethyl ist,
wobei das Verfahren entweder

a) die Umsetzung eines 4-Halogen-3-nitroanilins der Formel (9), wie in Anspruch 4 definiert, mit einem Alkylsulfonylhalogenid der Formel (11), wie in Anspruch 4 definiert, umfaßt, wobei ein Nitroanilinderivat der Formel (1) erhalten wird; oder

b) die Reduktion eines Nitroanilinderivats der Formel (1), wie in Anspruch 3 definiert, umfaßt, wobei ein Diaminderivat der Formel (2) erhalten wird.

**10.** Nitrocarbanilidderivat der Formel (3), wie in Anspruch 6 definiert, mit der Maßgabe, daß, falls X Fluor oder Chlor ist, R' nicht Methyl ist, falls X Fluor ist, R' nicht Phenyl ist, und, falls X Chlor ist, R' nicht Ethyl ist;
Aminocarbanilidderivat der Formel (4), wie in Anspruch 5 definiert, mit der Maßgabe, daß, falls X Fluor oder Chlor ist, R' nicht Methyl ist, falls X Fluor ist, R' nicht Phenyl ist, und, falls X Chlor ist, R' nicht Ethyl ist;
Anilidderivat der Formel (5), wie in Anspruch 1 definiert, mit der Maßgabe, daß falls X Chlor und R Trifluormethyl ist, R' nicht Ethyl ist; und
Sulfonamidderivat der Formel (6) wie in Anspruch 1 definiert.

**11.** Verfahren zur Herstellung eines Nitrocarbanilidderivats der Formel (3), wie in Anspruch 6 definiert,
eines Aminocarbanilidderivats der Formel (4), wie in Anspruch 5 definiert,
eines Anilidderivats der Formel (5) wie in Anspruch 1 definiert, mit der Maßgabe, daß, falls X Chlor und R Trifluormethyl ist, R' nicht Ethyl ist, oder
eines Sulfonamidderivats der Formel (6), wie in Anspruch 1 definiert,
wobei das Verfahren

(c) die Umsetzung eines 2-Halogen-5-nitroanilins der Formel (10), wie in Anspruch 7 definiert, mit einem Halogenformiat der Formel (12), wie in Anspruch 7 definiert, umfaßt, wobei ein Nitrocarbanilidderivat der Formel (3) erhalten wird;

(d) die Umsetzung eines Nitrocarbanilidderivats der Formel (3), wie in Anspruch 6 definiert, umfaßt, wobei ein Aminocarbanilidderivat der Formel (4) erhalten wird;

(e) die Umsetzung eines Aminocarbanilidderivats der Formel (4), wie in Anspruch 5 definiert, mit einem Alkylsulfonylhalogenid der Formel (11), wie in Anspruch 4 definiert, umfaßt, wobei ein Anilidderivat der Formel (5) erhalten wird; oder

(f) die Halogenierung eines Anilidderivats der Formel (5), wie in Anspruch 1 definiert, umfaßt, wobei ein Sulfonamidderivat der Formel (6) erhalten wird.

**12.** Verfahren zur Herstellung eines 6-Halogenalkyl-3-phenyluracilderivats der Formel (8):

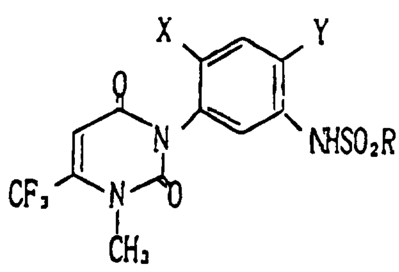

(8) ,

worin X, Y und R wie in Anspruch 1 definiert sind, wobei das Verfahren entweder

(a) die Umsetzung eines Sulfonamidderivats der Formel (6), wie in Anspruch 1 definiert, mit einer Verbindung der Formel (14) umfaßt:

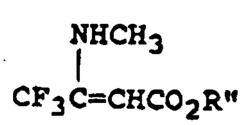

(14),

worin R'' $C_1$-$C_6$-Alkyl, Phenyl oder Benzyl ist, oder

(b) die Umsetzung eines Sulfonamidderivats der Formel (6), wie in Anspruch 1 definiert, mit einer Verbindung der Formel (13):

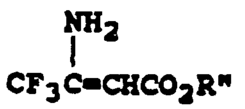

(13),

worin R'' wie oben definiert ist, wobei ein Zwischenprodukt der Formel (7) hergestellt wird:

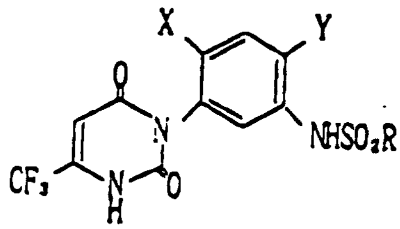

(7),

worin X, Y und R wie in Anspruch 1 definiert sind, und die Methylierung der Verbindung der Formel (7) umfaßt.

**13.** Verfahren gemäß Anspruch 12, das zusätzlich die Formulierung der so erhaltenen Verbindung der Formel (8) mit einem für Herbizide geeigneten Träger oder Verdünnungsmittel umfaßt.

**Patentansprüche für folgenden Vertragsstaat : ES**

**1.** Verfahren zur Herstellung eines Sulfonamidderivats der Formel (6):

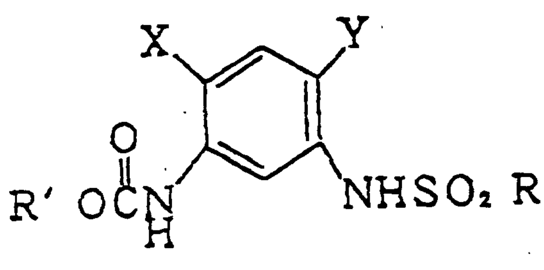

(6),

worin X und Y jeweils unabhängig ein Halogenatom sind, R $C_1$-$C_4$-Alkyl oder $C_1$-$C_3$-Halogenalkyl ist, und R' $C_1$-$C_4$-Alkyl oder Phenyl ist, wobei das Verfahren die Halogenierung eines Anilidderivats der Formel (5) umfaßt:

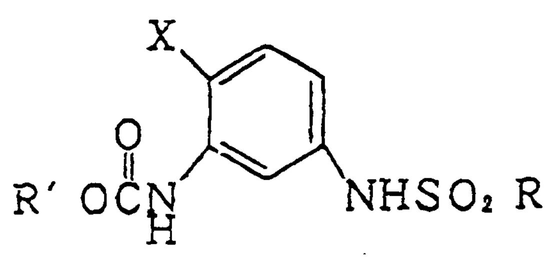

(5),

worin X, R und R' wie oben definiert sind.

2. Verfahren gemäß Anspruch 1, worin das Anilidderivat der Formel (5) hergestellt wird durch Umsetzung eines Diaminderivats der Formel (2):

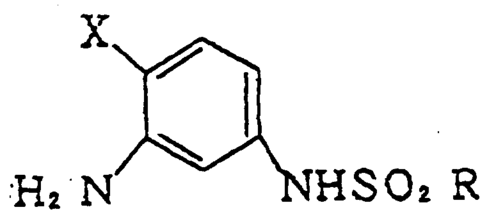

(2),

worin X und R wie in Anspruch 1 definiert sind,
mit einem Halogenformiat der Formel (12):

Hal—$CO_2R'$ (12),

worin Hal ein Halogenatom darstellt, und R' wie in Anspruch 1 definiert ist.

3. Verfahren gemäß Anspruch 2, worin das Diaminderivat der Formel (2) hergestellt wird durch Reduktion eines Nitroanilinderivats der Formel (1):

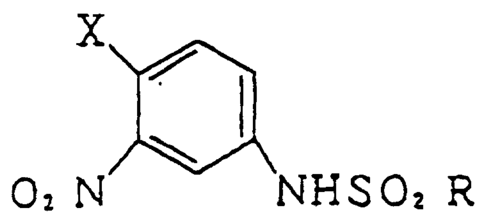

(1),

worin X und R wie in Anspruch 1 definiert sind.

4. Verfahren gemäß Anspruch 3, worin das Nitroanilinderivat der Formel (1) hergestellt wird durch Umsetzung eines 4-Halogen-3-nitroanilins der Formel (9):

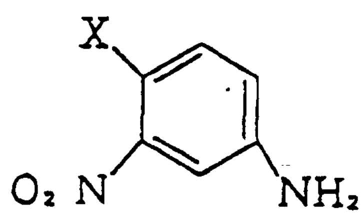

(9),

worin X wie in Anspruch 1 definiert ist,
mit einem Alkylsulfonylhalogenid der Formel (11):

Hal—$SO_2R$ (11)

worin Hal ein Halogenatom darstellt und R wie in Anspruch 1 definiert ist.

5. Verfahren gemäß Anspruch 1, worin das Anilidderivat der Formel (5) hergestellt wird durch Umsetzung eines Aminocarbanilidderivats der Formel (4):

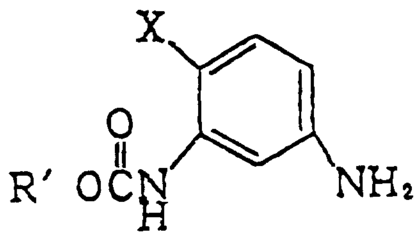

(4) ,

worin X und R' wie in Anspruch 1 definiert sind, mit einem Alkylsulfonylhalogenid der Formel (11), wie in Anspruch 4 definiert.

**6.** Verfahren gemäß Anspruch 5, worin das Aminocarbanilidderivat der Formel (4) hergestellt wird durch Reduktion eines Nitrocarbanilidderivats der Formel (3):

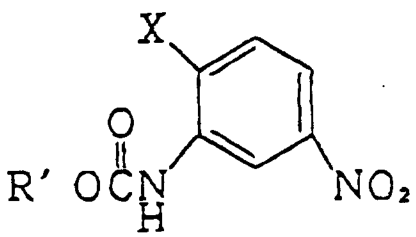

(3) ,

worin R' und X wie in Anspruch 1 definiert sind.

**7.** Verfahren gemäß Anspruch 6, worin das Nitrocarbanilidderivat der Formel (3) hergestellt wird durch Umsetzung eines 2-Halogen-5-nitroanilins der Formel (10):

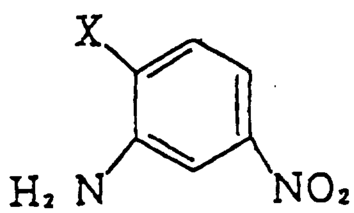

(10) ,

worin X wie in Anspruch 1 definiert ist, mit einem Halogenformiat der Formel (12), wie in Anspruch 2 definiert.

**8.** Verfahren zur Herstellung eines Nitroanilinderivats der Formel (1), wie in Anspruch 3 definiert, mit der Maßgabe, daß, falls X Chlor ist, R nicht Methyl, Chlormethyl oder Trifluormethyl ist,
oder eines Diaminderivats der Formel (2), wie in Anspruch 2 definiert, mit der Maßgabe, daß, falls X Chlor ist, R nicht Trifluormethyl ist,
wobei das Verfahren entweder

a) die Umsetzung eines 4-Halogen-3-nitroanilins der Formel (9), wie in Anspruch 4 definiert, mit einem Alkylsulfonylhalogenid der Formel (11), wie in Anspruch 4 definiert, umfaßt, wobei ein Nitroanilinderivat der Formel (1) erhalten wird; oder

b) die Reduktion eines Nitroanilinderivats der Formel (1), wie in Anspruch 3 definiert, umfaßt, wobei ein Diaminderivat der Formel (2) erhalten wird.

**9.** Verfahren zur Herstellung eines Nitrocarbanilidderivats der Formel (3), wie in Anspruch 6 definiert,
eines Aminocarbanilidderivats der Formel (4), wie in Anspruch 5 definiert,
eines Anilidderivats der Formel (5), wie in Anspruch 1 definiert, mit der Maßgabe, daß, falls X Chlor und R Trifluormethyl ist, R' nicht Ethyl ist, oder
eines Sulfonamidderivats der Formel (6), wie in Anspruch 1 definiert,
wobei das Verfahren

(c) die Umsetzung eines 2-Halogen-5-nitroanilins der Formel (10), wie in Anspruch 7 definiert, mit einem Halogenformiat der Formel (12), wie in Anspruch 7 definiert, umfaßt, wobei ein Nitrocarbanilidderivat der Formel (3) erhalten wird;
(d) die Umsetzung eines Nitrocarbanilidderivats der Formel (3), wie in Anspruch 6 definiert, umfaßt, wobei ein Aminocarbanilidderivat der Formel (4) erhalten wird;
(e) die Umsetzung eines Aminocarbanilidderivats der Formel (4), wie in Anspruch 5 definiert, mit einem Alkylsulfonylhalogenid der Formel (11), wie in Anspruch 4 definiert, umfaßt, wobei ein Anilidderivat der Formel (5) erhalten wird; oder
(f) die Halogenierung eines Anilidderivats der Formel (5), wie in Anspruch 1 definiert, umfaßt, wobei ein Sulfonamidderivat der Formel (6) erhalten wird.

**10.** Verfahren zur Herstellung eines 6-Halogenalkyl-3-phenyluracilderivats der Formel (8):

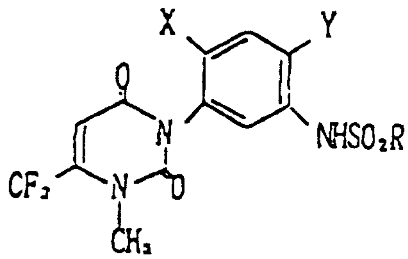

(8),

worin X, Y und R wie in Anspruch 1 definiert sind,
wobei das Verfahren entweder
(a) die Umsetzung eines Sulfonamidderivats der Formel (6), wie in Anspruch 1 definiert, mit einer Verbindung der Formel (14) umfaßt:

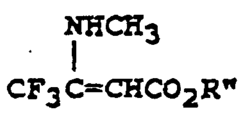

(14),

worin R'' $C_1$-$C_6$-Alkyl, Phenyl oder Benzyl ist, oder
(b) die Umsetzung eines Sulfonamidderivats der Formel (6), wie in Anspruch 1 definiert, mit einer Verbindung der Formel (13):

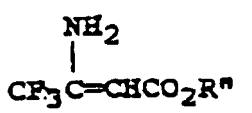

(13),

worin R'' wie oben definiert ist, wobei ein Zwischenprodukt der Formel (7) hergestellt wird:

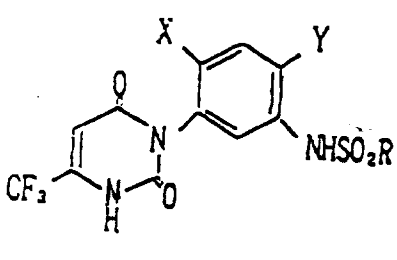

(7),

worin X, Y und R wie in Anspruch 1 definiert sind, und die Methylierung der Verbindung der Formel (7) umfaßt.

11. Verfahren gemäß Anspruch 10, das zusätzlich die Formulierung der so erhaltenen Verbindung der Formel (8) mit einem für Herbizide geeigneten Träger oder Verdünnungsmittel umfaßt.

**Revendications**

**Revendications pour les Etats contractants suivants : GB, DE, FR, IT, NL, CH, BE, AT, SE, GR, LU, DK, MC, PT, LI**

1. Procédé pour produire un dérivé de sulfonamide de formule (6) :

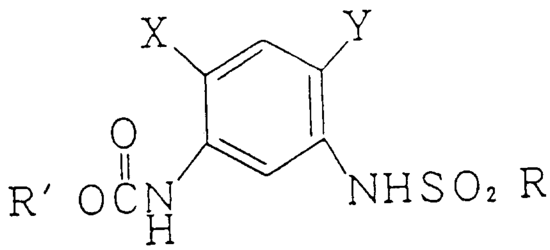

dans laquelle X et Y, indépendamment l'un de l'autre, sont chacun un atome d'halogène, R est un radical alkyle en $C_1$-$C_4$ ou halogènalkyle en $C_1$-$C_3$, et R' est un radical alkyle en $C_1$-$C_4$ ou phényle, procédé qui consiste à halogéner un dérivé d'anilide de formule (5) :

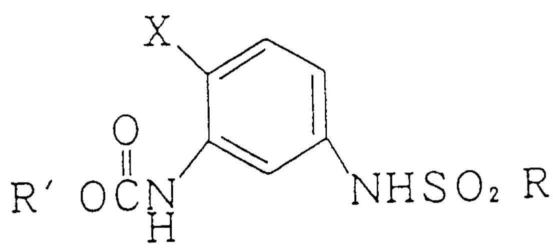

dans laquelle X, R et R' sont tels que définis ci-dessus.

2. Procédé selon la revendication 1, dans lequel le dérivé d'anilide de formule (5) est produit par la réaction d'un dérivé de diamine de formule (2) :

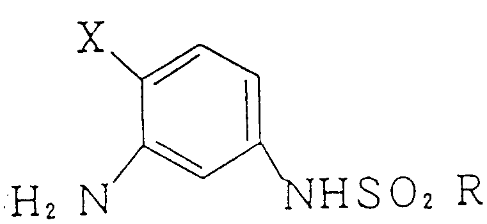

dans laquelle X et R sont tels que définis ci-dessus, avec un halogénoformiate de formule (12) :

$Hal—CO_2R'$ (12)

dans laquelle Hal représente un atome d'halogène et R' est tel que défini dans la revendication 1.

3. Procédé selon la revendication 2, dans lequel le dérivé de diamine de formule (2) est produit par la réduction d'un dérivé de nitroaniline de formule (1) :

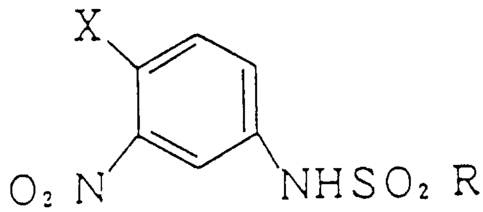

dans laquelle X et R sont tels que définis dans la revendication 1.

**4.** Procédé selon la revendication 3, dans lequel le dérivé de nitroaniline de formule (1) est produit par la réaction d'une 4-halogéno-3-nitroaniline de formule (9) :

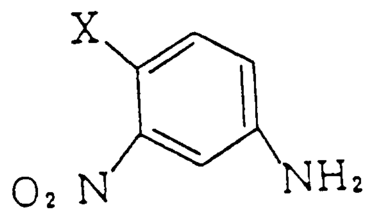

dans laquelle X est tel que défini dans la revendication 1, avec un halogénure d'alkylsulfonyle de formule (11) :

Hal—$SO_2$R (11)

dans laquelle Hal représente un atome d'halogène et R est tel que défini dans la revendication 1.

**5.** Procédé selon la revendication 1, dans lequel le dérivé d'anilide de formule (5) est produit par la réaction d'un dérivé d'aminocarbanilide de formule (4) :

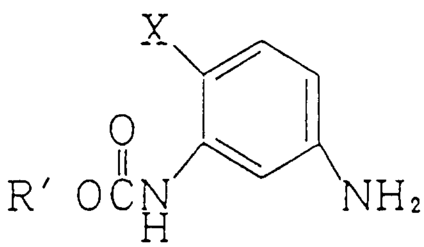

dans laquelle X et R' sont tels que définis dans la revendication 1, avec un halogénure d'alkylsulfonyle de formule (11), tel que défini dans la revendication 4.

**6.** Procédé selon la revendication 5, dans lequel le dérivé d'aminocarbanilide de formule (4) est produit par la réduction d'un dérivé de nitrocarbanilide de formule (3) :

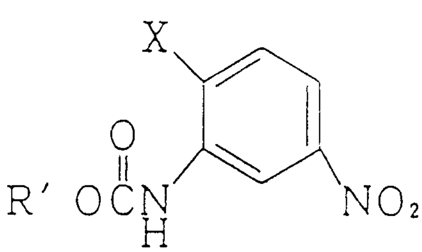

dans laquelle R' et X sont tels que définis dans la revendication 1.

7. Procédé selon la revendication 6, dans lequel le dérivé de nitrocarbanilide de formule (3) est produit par la réaction d'une 2-halogéno-5-nitroaniline de formule (10) :

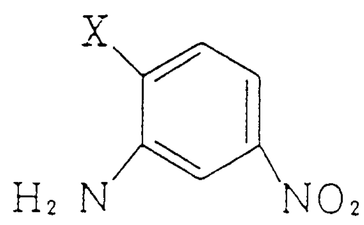

dans laquelle X est tel que défini dans la revendication 1, avec un halogénoformiate de formule (12) selon la revendication 2.

8. Dérivé de nitroaniline de formule (1) selon la revendication 3, du moment que, quand X est un fluor, R n'est pas le radical méthyle, et, quand X est le chlore, R n'est pas le radical méthyle, chlorométhyle ou trifluorométhyle ; et dérivé de diamine de formule (2) selon la revendication 2, du moment que, quand X est le chlore, R n'est pas le radical méthyle ou trifluorométhyle.

9. Procédé pour produire un dérivé de nitroaniline de formule (1) selon la revendication 3, du moment que, quand X est le chlore, R n'est pas le radical méthyle, chlorométhyle ou trifluorométhyle, ou d'un dérivé de diamine de formule (2) selon la revendication 2, du moment que, quand X est le chlore, R n'est pas le radical trifluorométhyle, lequel procédé consiste :
   (a) à faire réagir une 4-halogéno-3-nitroaniline de formule (9) selon la revendication 4, avec un halogénure d'alkylsulfonyle de formule (11) selon la revendication 4, pour obtenir ledit dérivé de nitroaniline de formule (1) ; ou bien
   (b) à réduire un dérivé de nitroaniline de formule (1) selon la revendication 3, pour obtenir ledit dérivé de diamine de formule (2).

10. Dérivé de nitrocarbanilide de formule (3) selon la revendication 6, du moment que, quand X est le fluor ou le chlore, R' n'est pas le radical méthyle ; quand X est le fluor, R' n'est pas le radical phényle ; et quand X est le chlore, R' n'est pas le radical phényle ; et quand X est le chlore, R' n'est pas le radical éthyle ; dérivé d'aminocarbanilide de formule (4) selon la revendication 5, du moment que, quand X est le fluor ou le chlore, R' n'est pas le radical méthyle ; quand X est le fluor, R' n'est pas le radical phényle ; et quand X est le chlore, R' n'est pas le radical éthyle ; dérivé d'anilide de formule (5) selon la revendication 1, du moment que, quand X est le chlore et R est le radical trifluorométhyle, R' n'est pas le radical éthyle ; et dérivé de sulfonamide de formule (6) selon la revendication 1.

11. Procédé pour produire un dérivé de nitrocarbanilide de formule (3) selon la revendication 6, un dérivé d'aminocarbanilide de formule (4) selon la revendication 5, un dérivé d'anilide de formule (5) selon la revendication 1, du moment que, quand x est le chlore et R est le radical trifluorométhyle, R' n'est pas le radical éthyle, ou bien dérivé de sulfonamide de formule (6) selon la revendication 1, lequel procédé consiste :
   (c) à faire réagir une 2-halogéno-5-nitroaniline de formule (10) selon la revendication 7, avec un halogénoformiate de formule (12) selon la revendication 7, pour obtenir ledit dérivé de nitrocarbanilide de formule (3) ;
   (d) à réduire un dérivé de nitrocarbanilide de formule (3) selon la revendication 6, pour obtenir ledit dérivé d'aminocarbanilide de formule (4) ;
   (e) à faire réagir un dérivé d'aminocarbanilide de formule (4) selon la revendication 5, avec un halogénure d'alkylsulfonyle de formule (11) selon la revendication 4, pour obtenir ledit dérivé d'aniline de formule (5) ; ou bien
   (f) à halogéner un dérivé d'anilide de formule (5) selon la revendication 1, pour obtenir ledit dérivé de sulfonamide de formule (6).

**12.** Procédé pour produire un dérivé de 6-halogènalkyl-3-phényluracile de formule (8)

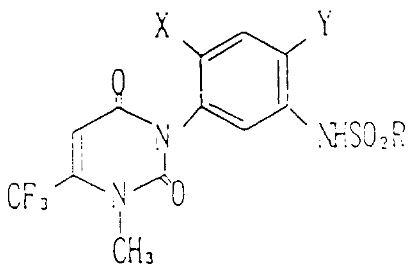

dans laquelle X, Y et R sont tels que définis dans la revendication 1, lequel procédé consiste :

(a) à faire réagir un dérivé de sulfonamide de formule (6) selon la revendication 1, avec un composé de formule (14)

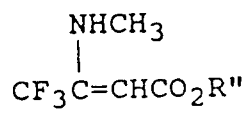

dans laquelle R" est un radical alkyle en $C_1$-$C_6$, phényle ou benzyle, ou bien

(b) à faire réagir un dérivé de sulfonamide de formule (6) selon la revendication 1, avec un composé de formule (13) :

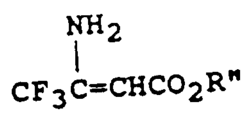

dans laquelle R" est tel que défini ci-dessus, pour produire un composé intermédiaire de formule (7) :

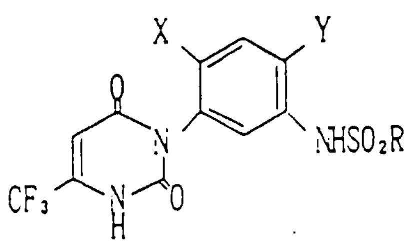

dans laquelle X, Y et R sont tels que définis dans la revendication 1, et à méthyler ce composé de formule (7).

**13.** Procédé selon la revendication 12, qui consiste, en outre, à formuler le composé de formule (8) ainsi obtenu avec un support ou un diluant acceptable d'un point de vue herbicide.

**Revendications pour l'Etat contractant suivant : ES**

1. Procédé pour produire un dérivé de sulfonamide de formule (6) :

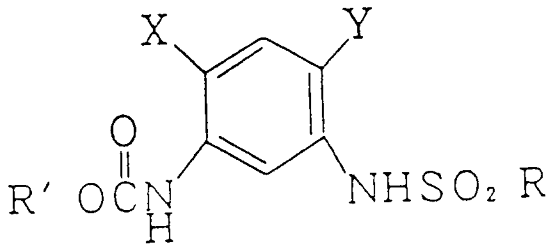

dans laquelle X et Y, indépendamment l'un de l'autre, sont chacun un atome d'halogène, R est un radical alkyle en $C_1$-$C_4$ ou halogènalkyle en $C_1$-$C_3$, et R' est un radical alkyle en $C_1$-$C_4$ ou phényle, procédé qui consiste à halogéner un dérivé d'anilide de formule (5) :

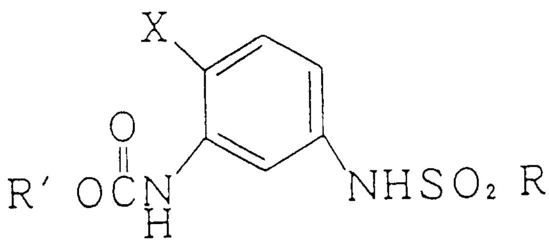

dans laquelle X, R et R' sont tels que définis ci-dessus.

2. Procédé selon la revendication 1, dans lequel le dérivé d'anilide de formule (5) est produit par la réaction d'un dérivé de diamine de formule (2) :

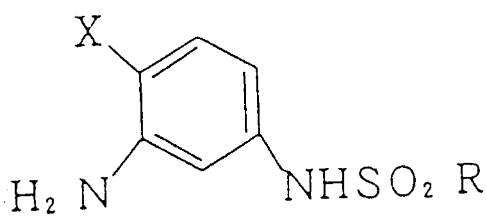

dans laquelle X et R sont tels que définis ci-dessus, avec un halogénoformiate de formule (12) :

$Hal—CO_2R'$ (12)

dans laquelle Hal représente un atome d'halogène et R' est tel que défini dans la revendication 1.

3. Procédé selon la revendication 2, dans lequel le dérivé de diamine de formule (2) est produit par la réduction d'un dérivé de nitroaniline de formule (1) :

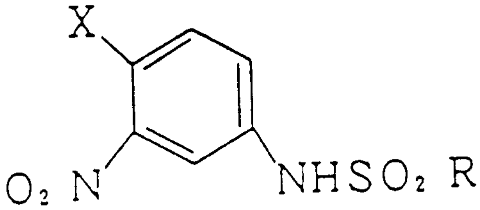

dans laquelle X et R sont tels que définis dans la revendication 1.

**4.** Procédé selon la revendication 3, dans lequel le dérivé de nitroaniline de formule (1) est produit par la réaction d'une 4-halogéno-3-nitroaniline de formule (9) :

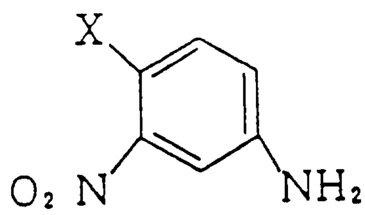

dans laquelle X est tel que défini dans la revendication 1, avec un halogénure d'alkylsulfonyle de formule (11) :

Hal—$SO_2$R (11)

dans laquelle Hal représente un atome d'halogène et R est tel que défini dans la revendication 1.

**5.** Procédé selon la revendication 1, dans lequel le dérivé d'anilide de formule (5) est produit par la réaction d'un dérivé d'aminocarbanilide de formule (4) :

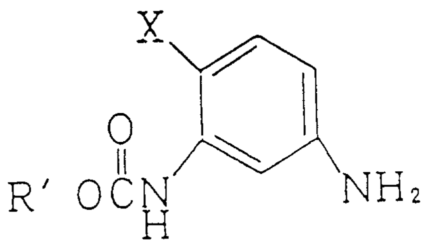

dans laquelle X et R' sont tels que définis dans la revendication 1, avec un halogénure d'alkylsulfonyle de formule (11), tel que défini dans la revendication 4.

**6.** Procédé selon la revendication 5, dans lequel le dérivé d'aminocarbanilide de formule (4) est produit par la réduction d'un dérivé de nitrocarbanilide de formule (3) :

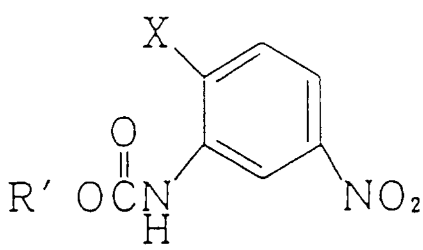

dans laquelle R' et X sont tels que définis dans la revendication 1.

**7.** Procédé selon la revendication 6, dans lequel le dérivé de nitrocarbanilide de formule (3) est produit par la réaction d'une 2-halogéno-5-nitroaniline de formule (10) :

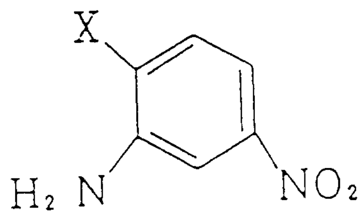

dans laquelle X est tel que défini dans la revendication 1, avec un halogénoformiate de formule (12) selon la revendication 2.

**8.** Procédé pour produire un dérivé de nitroaniline de formule (1) selon la revendication 3, du moment que, quand X est le chlore, R n'est pas le radical méthyle, chlorométhyle ou trifluorométhyle, ou d'un dérivé de diamine de formule (2) selon la revendication 2, du moment que, quand X est le chlore, R n'est pas le radical trifluorométhyle, lequel procédé consiste :
(a) à faire réagir une 4-halogéno-3-nitroaniline de formule (9) selon la revendication 4, avec un halogénure d'alkylsulfonyle de formule (11) selon la revendication 4, pour obtenir ledit dérivé de nitroaniline de formule (1) ; ou bien
(b) à réduire un dérivé de nitroaniline de formule (1) selon la revendication 3, pour obtenir ledit dérivé de diamine de formule (2).

**9.** Procédé pour produire un dérivé de nitrocarbanilide de formule (3) selon la revendication 6, un dérivé d'aminocarbanilide de formule (4) selon la revendication 5, un dérivé d'anilide de formule (5) selon la revendication 1, du moment que, quand x est le chlore et R est le radical trifluorométhyle, R' n'est pas le radical éthyle, ou bien dérivé de sulfonamide de formule (6) selon la revendication 1, lequel procédé consiste :
(c) à faire réagir une 2-halogéno-5-nitroaniline de formule (10) selon la revendication 7, avec un halogénoformiate de formule (12) selon la revendication 7, pour obtenir ledit dérivé de nitrocarbanilide de formule (3) ;
(d) à réduire un dérivé de nitrocarbanilide de formule (3) selon la revendication 6, pour obtenir ledit dérivé d'aminocarbanilide de formule (4) ;
(e) à faire réagir un dérivé d'aminocarbanilide de formule (4) selon la revendication 5, avec un halogénure d'alkylsulfonyle de formule (11) selon la revendication 4, pour obtenir ledit dérivé d'aniline de formule (5) ; ou bien
(f) à halogéner un dérivé d'anilide de formule (5) selon la revendication 1, pour obtenir ledit dérivé de sulfonamide de formule (6).

**10.** Procédé pour produire un dérivé de 6-halogènalkyl-3-phényluracile de formule (8)

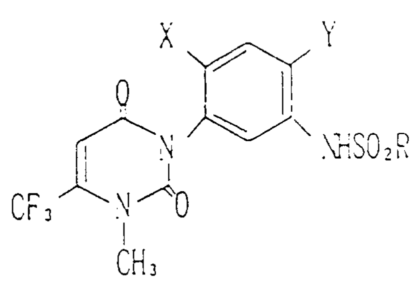

dans laquelle X, Y et R sont tels que définis dans la revendication 1, lequel procédé consiste :
(a) à faire réagir un dérivé de sulfonamide de formule (6) selon la revendication 1, avec un composé de formule (14)

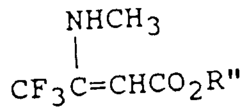

dans laquelle R'' est un radical alkyle en $C_1$-$C_6$, phényle ou benzyle, ou bien

(b) à faire réagir un dérivé de sulfonamide de formule (6) selon la revendication 1, avec un composé de formule (13) :

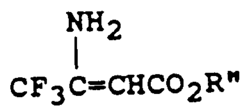

dans laquelle R'' est tel que défini ci-dessus, pour produire un composé intermédiaire de formule (7) :

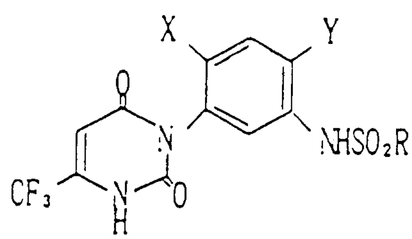

dans laquelle X, Y et R sont tels que définis dans la revendication 1, et à méthyler ce composé de formule (7).

**11.** Procédé selon la revendication 12, qui consiste, en outre, à formuler le composé de formule (8) ainsi obtenu avec un support ou un diluant acceptable d'un point de vue herbicide.